# EUROPEAN PATENT SPECIFICATION

(11) **EP 3 065 770 B1**
(45) Date of publication and mention of the grant of the patent: **28.10.2020**
(21) Application number: 14803277.4
(22) Date of filing: 31.10.2014
(51) Int. Cl.: A61K 39/00, A61P 35/00

(54) **COMBINATION THERAPY FOR TREATING CANCER WITH A POXVIRUS EXPRESSING A TUMOR ANTIGEN AND AN ANTAGONIST OF AN IMMUNE CHECKPOINT INHIBITOR**
KOMBINATIONSTHERAPIE ZUR BEHANDLUNG VON KREBS MIT EINEM EIN TUMOR-ANTIGEN EXPRIMIERENDEN POCKENVIRUS UND EINEM ANTAGONISTEN EINES IMMUN-CHECKPOINT-HEMMERS
POLYTHÉRAPIE POUR LE TRAITEMENT DU CANCER À L'AIDE D'UN POXVIRUS EXPRIMANT UN ANTIGÈNE TUMORAL ET D'UN ANTAGONISTE D'UN INHIBITEUR DE POINT DE CONTRÔLE IMMUNITAIRE

(30) Priority: 05.11.2013 US 201361900226 P
(43) Date of publication of application: 14.09.2016
(73) Proprietor: Bavarian Nordic A/S, 3490 Kvistgaard (DK)
(72) Inventor: ROUNTREE, Ryan, San Jose, CA 95118 (US); FOY, Susan, Mountain View, CA 94041 (US); MANDL, Stefanie, San Francisco, CA 94122 (US); DELCAYRE, Alain, San Jose, CA 95124 (US)
(74) Representative: Bendiksen, Henrik
(86) International application number: PCT/US2014/063516
(87) International publication number: WO 2015/069571

(56) References cited:
- WO-A1-98/04727
- WO-A1-2010/050913
- WO-A2-2011/041613
- RAVI A. MADAN ET AL.: "Ipilimumab and a poxviral vaccine targeting prostate-specific antigen in metastatic castration-resistant prostate cancer: a phase 1 dose-escalation trial", LANCET ONCOLOGY, vol. 13, no. 5, 10 February 2012 (2012-02-10), pages 501-508, XP002735511,
- MADAN RAVI A ET AL: "Combination of vaccine and immune checkpoint inhibitor is safe with encouraging clinical activity", ONCOIMMUNOLOGY, vol. 1, no. 7, October 2012 (2012-10), pages 1167-1168, XP002735512,
- S.-R. WOO ET AL: "Immune Inhibitory Molecules LAG-3 and PD-1 Synergistically Regulate T-cell Function to Promote Tumoral Immune Escape", CANCER RESEARCH, vol. 72, no. 4, 20 December 2011 (2011-12-20), pages 917-927, XP055151722, ISSN: 0008-5472, DOI: 10.1158/0008-5472.CAN-11-1620 cited in the application
- STEFANIE J MANDL ET AL: "Immunotherapy with MVA-BN-HER2 induces HER-2-specific Th1 immunity and alters the intratumoral balance of effector and regulatory T cells", CANCER IMMUNOLOGY, IMMUNOTHERAPY, SPRINGER, BERLIN, DE, vol. 61, no. 1, 7 August 2011 (2011-08-07) , pages 19-29, XP019995547, ISSN: 1432-0851, DOI: 10.1007/S00262-011-1077-4 cited in the application
- SCHLOM JEFFREY: "Therapeutic Cancer Vaccines: Current Status and Moving Forward", JOURNAL OF THE NATIONAL CANCER INSTITUTE (CARY), vol. 104, no. 8, April 2012 (2012-04), pages 599-613, XP002735513,
- GULLEY JAMES L ET AL: "Pilot study of vaccination with recombinant CEA-MUC-1-TRICOM poxviral-based vaccines in patients with metastatic carcinoma", CLINICAL CANCER RESEARCH, AMERICAN ASSOCIATION FOR CANCER RESEARCH, US, vol. 14, no. 10, 15 May 2008 (2008-05-15), pages 3060-3069, XP009104603, ISSN: 1078-0432, DOI: 10.1158/1078-0432.CCR-08-0126

## Description

### FIELD OF THE INVENTION

The invention relates to the treatment of cancers using recombinant poxviruses encoding a tumor antigen. More particularly, the present invention is directed to the treatment of cancers using one or more recombinant poxviruses encoding a tumor antigen in combination with one or more agonists and/or antagonists of an immune checkpoint inhibitor.

### BACKGROUND OF THE INVENTION

Recombinant poxviruses have been used as vaccines for infectious organisms and, more recently, for tumors. Mastrangelo et al. J Clin Invest. 2000;105(8): 1031-1034. Two of these poxvirus groups, avipoxvirus and orthopoxvirus, have been shown to be effective at battling tumors and have been involved with potential cancer treatments.

One exemplary avipoxvirus species, fowlpox, has been shown to be a safe vehicle for human administrations as fowlpox virus enters mammalian cells and expresses proteins, but replicates abortively. Skinner et al. Expert Rev Vaccines. 2005 Feb;4(1):63-76. Additionally, the use of fowlpox virus as a vehicle for expression is being evaluated in numerous clinical trials of vaccines against cancer, malaria, tuberculosis, and AIDS. *Id.*

Orthopoxviruses have been shown to be useful vectors for the administration of antigens to patients to induce an immune response against the antigen. Vaccinia, the most well-known of the orthopoxviruses, was used in the world-wide eradication of smallpox and has shown usefulness as a vector and/or vaccine. Recombinant Vaccinia Vector has been engineered to express a wide range of inserted genes, including several tumor associated genes such as p97, HER-2/neu, p53 and ETA (Paoletti, et al., 1993).

Another known orthopoxvirus is Modified Vaccinia Ankara (MVA) virus. MVA is related to vaccinia virus, a member of the genera Orthopoxvirus, in the family of Poxviridae. MVA was generated by 516 serial passages on chicken embryo fibroblasts of the Ankara strain of vaccinia virus (CVA) (for review see Mayr, A., et al. Infection 3, 6-14 (1975)). As a consequence of these long-term passages, the genome of the resulting MVA virus had about 31 kilobases of its genomic sequence deleted and, therefore, was described as highly host cell restricted for replication to avian cells (Meyer, H. et al., J. Gen. Virol. 72, 1031-1038 (1991)). It was shown in a variety of animal models that the resulting MVA was significantly avirulent (Mayr, A. & Danner, K., Dev. Biol. Stand. 41: 225-34 (1978)). Additionally, this MVA strain has been tested in clinical trials as a vaccine to immunize against the human smallpox disease (Mayr et al., Zbl. Bakt. Hyg. I, Abt. Org. B 167, 375-390 (1987); Stickl et al., Dtsch. med. Wschr. 99, 2386-2392 (1974)). In these human studies, MVA had diminished virulence or infectiousness as compared to vaccinia-based vaccines, while MVA still induced a good specific immune response.

In the following decades, MVA was engineered for use as a viral vector for recombinant gene expression or as a recombinant vaccine (Sutter, G. et al., Vaccine 12: 1032-40 (1994)).

Even though Mayr et al. demonstrated during the 1970s that MVA is highly attenuated and avirulent in humans and mammals, certain investigators have reported that MVA is not fully attenuated in mammalian and human cell lines since residual replication might occur in these cells. (Blanchard et al., J Gen Virol 79, 1159-1167 (1998); Carroll & Moss, Virology 238, 198-211 (1997); Altenberger, U.S. Pat. No. 5,185,146; Ambrosini et al., J Neurosci Res 55(5), 569 (1999)). It is assumed that the results reported in these publications have been obtained with various known strains of MVA, since the viruses used essentially differ in their properties, particularly in their growth behavior in various cell lines. Such residual replication is undesirable for various reasons, including safety concerns in connection with use in humans.

Strains of MVA having enhanced safety profiles for the development of safer products, such as vaccines or pharmaceuticals, have been described. *See* U.S. Pat. Nos. 6,761,893 and 6,193,752. Such strains are capable of reproductive replication in non-human cells and cell lines, especially in chicken embryo fibroblasts (CEF), but are not capable of significant reproductive replication in certain human cell lines known to permit replication with known vaccinia strains. Such cell lines include a human keratinocyte cell line, HaCat (Boukamp et al. J Cell Biol 106(3): 761-71 (1988)), a human cervix adenocarcinoma cell line, HeLa (ATCC No. CCL-2), a human embryo kidney cell line, 293 (ECACC No. 85120602), and a human bone osteosarcoma cell line, 143B (ECACC No. 91112502). Such strains are also not capable of significant reproductive replication *in vivo,* for example, in certain mouse strains, such as the transgenic mouse model AGR 129, which is severely immune-compromised and highly susceptible to a replicating virus. *See* U.S. Pat. Nos. 6,761,893. One such MVA strain and its derivatives and recombinants, referred to as "MVA-BN," has been described. *See* U.S. Pat. Nos. 6,761,893 and 6,193,752.

MVA and MVA-BN have each been engineered for use as a viral vector for recombinant gene expression or as a recombinant vaccine. *See, e.g.,* Sutter, G. et al., Vaccine 12: 1032-40 (1994), U.S. Pat. Nos. 6,761,893 and 6,193,752.

Certain approaches to cancer immunotherapy have included vaccination with tumor-associated antigens. In certain instances, such approaches have included use of a delivery system to promote host immune responses to tumor-associated antigens. In certain instances, such delivery systems have included recombinant viral vectors. *See, e.g.,* Harrop et al., Front. Biosci. 11:804-817 (2006); Arlen et al., Semin. Oncol. 32:549-555 (2005); Liu et al., Proc. Natl. Acad. Sci. USA 101 (suppl. 2):14567-14571 (2004).

HER-2 is a tumor-associated antigen that is over-expressed in tumor cells of a number of cancer patients. Immunization with various HER-2 polypeptides has been used to generate an immune response against tumor cells expressing this antigen. *See, e.g.,* Renard et al., J. Immunology 171:1588-1595 (2003); Mittendorf et al., Cancer 106:2309-2317 (2006).

An MVA encoding a HER-2 antigen, MVA-BN-HER2, has been shown to exert potent anti-tumor efficacy in a murine model of experimental pulmonary metastasis, despite a strong tumor-mediated immunosuppressive environment characterized by a high frequency of regulatory T cells (T_{reg}) in the lungs. Mandl et al., Cancer Immunol Immunother (2012) 61:19-29. The recombinant MVA was reported to induce strongly Th1-dominated HER-2-specific antibody and T-cell responses. *Id.* The anti-tumor activity was characterized by an increased infiltration of lungs with highly activated, HER-2-specific, CD8+CD11c+ T cells, and was accompanied by a decrease in the frequency of T_{reg} cells in the lung, resulting in a significantly increased ratio of effector T cells to T_{reg} cells. *Id.*

MVA-BN-HER2 has also been shown to be safe and break tolerance to induce specific T and B cell responses in human clinical studies in a metastatic setting. Guardino et al., Cancer Research: December 15, 2009; Volume 69, Issue 24, Supplement 3.

Trastuzumab (Herceptin) is a humanized monoclonal antibody (mAb) targeting the extra-cellular domain of HER2, and has shown clinical efficacy in HER2-positive breast cancer. Wang et al., Cancer Res. 2012 September 1; 72(17): 4417-4428. However, a significant number of patients fail to respond to initial trastuzumab treatment and many trastuzumab-responsive tumors develop resistance after continuous treatment. *Id.*

Inhibitory receptors on immune cells are pivotal regulators of immune escape in cancer. Woo et al., Cancer Res; 72(4); 917-27, 2011. Among these inhibitory receptors, CTLA-4 (cytotoxic T-lymphocyte-associated protein 4) serves as a dominant off-switch while other receptors such as PD-1 (programmed death 1, CD279) and LAG-3 (lymphocyte activation gene, CD223) seem to serve more subtle rheostat functions. *Id.*

CTLA-4 is an immune checkpoint molecule, which is up-regulated on activated T-cells. Mackiewicz, Wspolczesna onkol 2012; 16 (5):363-370. An anti-CTLA4 mAb can block the interaction of CTLA-4 with CD80/86 and switch off the mechanism of immune suppression and enable continuous stimulation of T-cells by DCs. Two IgG mAb directed against CTLA-4, ipilimumab and tremelimumab, have been used in clinical trials in patients with melanoma. However, treatments with anti-CTLA-4 antibodies have shown high levels of immune-related adverse events. *Id.*

Another human mAb modulating the immune system is BMS-936558 (MDX-1106) directed against the death-1 receptor (PD-1R), the ligand of which (PD-1L) can be directly expressed on melanoma cells. *Id.* PD-1R is a part of the B7:CD28 family of co-stimulatory molecules that regulate T-cell activation and tolerance, and thus anti-PD-1R can play a role in breaking tolerance. *Id.*

Engagement of the PD-1/PD-L1 pathway results in inhibition of T-cell effector function, cytokine secretion and proliferation. Turnis et al., OncoImmunology 1:7, 1172-1174; 2012. High levels of PD-1 are associated with exhausted or chronically stimulated T cells. *Id.* Moreover, increased PD-1 expression correlates with reduced survival in cancer patients. *Id.*

While these recent studies have suggested that PD-1 expression can be linked to survival rates in cancer, early studies with inhibition of PD-1 in treating cancers have shown a wide variety of adverse side effects. Mellman et al. Nature 2011; 480(7378): 480-489; see also Chow, Am Soc Clin Oncol Educ Book, 2013, "Exploring novel immune-related toxicities and endpoints with immune-checkpoint inhibitors in non-small cell lung cancer".

LAG-3 is a negative regulatory molecule expressed upon activation of various lymphoid cell types. *Id.* LAG-3 is required for the optimal function of both natural and induced immunosuppressive Treg cells. Id.

Combinatorial blockade of PD-1 and LAG-3 with monoclonal antibodies synergistically limited the growth of established tumors. Woo et al., Cancer Res; 72(4); 917-27, 2011. Although anti-LAG-3/anti-PD-1 combinatorial immunotherapy effectively cleared established Sa1N and MC38 tumors, this therapy was not effective against established B16 tumors. *Id.* Turnis et al. reported that their study, "highlighted the difficulty in predicting the outcome of combination treatments." Turnis et al., OncoImmunology 1:7, 1172-1174; 2012.

The inducible co-stimulatory molecule (ICOS) has been reported to be highly expressed on Tregs infiltrating various tumors, including melanoma and ovarian cancers. Faget et al., OncoImmunology 2:3, e23185; March 2013. It has also been reported that the ICOS/ICOSL interaction occurs during the interaction of TA-T_{regs} with TA-pDCs in breast carcinoma. *Id*. Antagonist antibodies against ICOS have been used to inhibit ICOS:ICOS-L interaction and abrogate proliferation of T_{reg} induced by pDC. WO 2012/131004. An antagonist antibody was used in a murine model of mammary tumor to reduce tumor progression. *Id.*

An agonist antibody directed against ICOS has been suggested as being useful in in combination with a blocking anti-CTLA-4 antibody and a blocking anti-PD-1 antibody for the treatment of tumors. WO 2011/041613.

The prior art also includes Gulley, James L et al., Clinical Cancer Research, American Association for Cancer Research, US, Vol. 14, no. 10, 15 May 2008, 3060-3069. This discloses the treatment of metastatic carcinoma patients comprising the administration of a CEA-MUC-1/TRICOM vaccine (PANVAC) which expresses T-cell costimulatory molecules B7.1, intercellular adhesion molecule 1 and Lymphocyte function associated antigen 3. However, no combination with PD-1 or LAG-3 antagonist antibody was dislosed.

There is clearly a substantial unmet medical need for additional cancer treatments, including active immunotherapies and cancer vaccines. In many aspects, the embodiments of the present disclosure address these needs by providing combination therapies that increase and improve the cancer treatments currently available.

### BRIEF SUMMARY OF THE INVENTION

In one general aspect, the present invention encompasses compositions for use in the treatment of a human cancer patients using recombinant poxviruses encoding at least one tumor antigen in combination with one or more antagonists or agonists of an immune checkpoint inhibitor.

In a more particular aspect, the present invention encompasses compositions utilizing a combination of an orthopoxvirus and/or an avipoxvirus expressing a tumor antigen with one or more combinations of antagonists of PD-1 and LAG-3.

In one embodiment, the present invention includes a medicament or composition for use in the treatment of a human cancer patient, comprising: (a) a first recombinant orthopoxvirus comprising a nucleic acid encoding a polypeptide of a CEA antigen and a nucleic acid encoding a polypeptide of a MUC-1 antigen; and (b) at least one of an antagonist of PD-1 that is an antibody and an antagonist of LAG-3 that is an antibody ; wherein (a) and (b) are to be administered as a combination.

In another embodiment, the present invention includes a medicament or composition for use in the treatment of a human cancer patient, further comprising a recombinant avipoxvirus comprising a nucleic acid encoding a polypeptide of at least one tumor antigen in combination with at least one of an anti-PD-1 antagonist that is an antibody and an anti-LAG-3 antagonist that is an antibody.

In still another embodiment, the medicament or composition for use in the treatment of a human cancer patient further comprises two or more recombinant orthopoxviruses comprising a nucleic acid encoding a polypeptide of at least one tumor antigen and at least one of an anti-PD-1 antagonist that is an antibody and/or an anti-LAG-3 antagonist that is an antibody.

In certain embodiments, as described herein, the recombinant orthopoxvirus is selected from a vaccinia virus, a modified vaccinia Ankara (MVA) virus, and/or MVA-BN. In certain additional embodiments, the recombinant avipoxvirus is a fowlpox virus.

In certain additional embodiments, the medicament or composition for use in the treatment of a human cancer patient encompasses a cancer selected from breast cancer, colorectal cancer, lung cancer, gastric cancer, pancreatic cancer, prostate cancer, bladder cancer, and/or ovarian cancer.

### BRIEF DESCRIPTION OF THE DRAWINGS

The accompanying drawings, which are incorporated in and constitute a part of this specification, illustrate one or more embodiments of the invention and together with the description, serve to explain the principles of the invention.

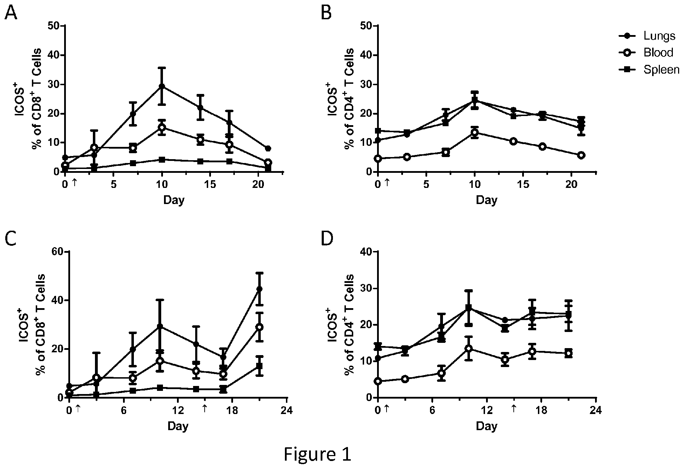

Figure 1. Shows ICOS expression in the lungs, spleen, and blood on T cells after treatment with MVA-BN-HER2. Naive (tumor-free) mice were treated on day 1 (A and B) or days 1 and 15 (C and D) with MVA-BN-HER2. ICOS expression on CD8+T cells (A and C) and CD4+ T cells (B and D). Data shown as mean ± SEM, three mice per group at each time point.

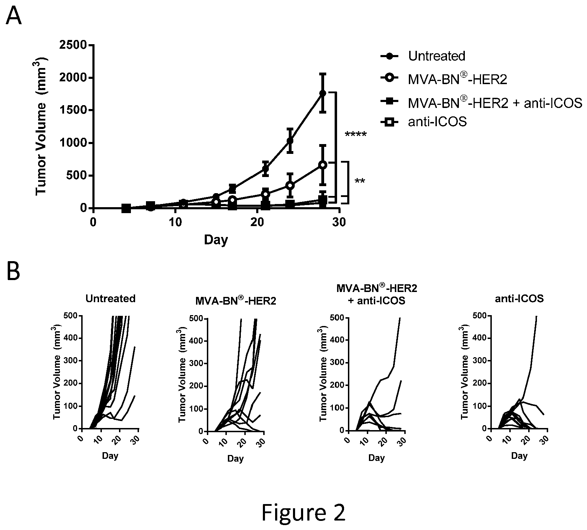

Figure 2. Shows tumor volume after administration of MVA-BN-HER2 in combination with anti-ICOS agonist. Mice with solid CT26-HER-2 tumors were treated with MVA-BN-HER2 on days 1 and 15, and anti-ICOS on days 1, 4, 8, 11, 15, 18, 22, 25 (i.p.). A) Average tumor growth. B) Tumor growth in individual mice.

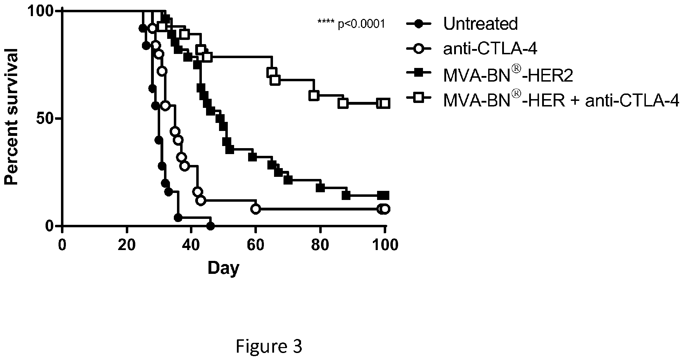

Figure 3. Shows MVA-BN-HER2 synergizes with anti-CTLA-4 to eliminate tumors and increase survival in an experimental lung metastasis model. Mice with CT26-HER-2 tumors in the lung were treated with MVA-BN-HER2 on days 4 and 18, and anti-CTLA-4 on days 3 and 17.

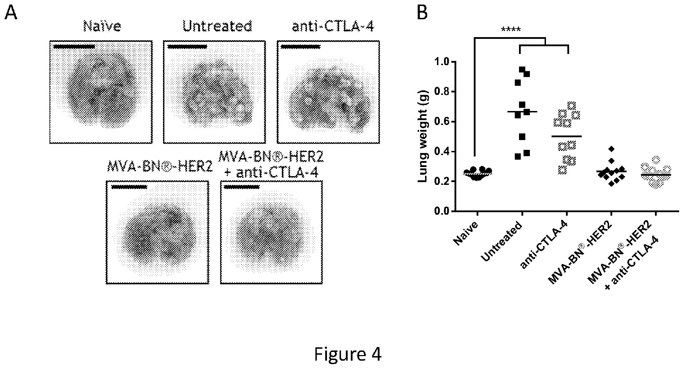

Figure 4. Shows MVA-BN-HER2 alone and in combination with anti-CTLA-4 reduces CT26-HER2 lung tumor burden 25 days after tumor implant. Mice with CT26-HER-2 tumors in the lung were treated with MVA-BN-HER2 on days 4 and 18, and anti-CTLA-4 on days 3 and 17; tumor burden analyzed on day 25. A) Representative lungs from each group show tumors visible as small masses in Untreated and anti-CTLA-4 treated lungs. There were no visible tumors in mice treated with MVA-BN-HER2. Scale bar equals 1 cm. B) Mice treated with MVA-BN-HER2 have similar lung weight to Naive mice on day 25, while lung weight is significantly greater in Untreated and anti-CTLA-4 treated mice on day 25.

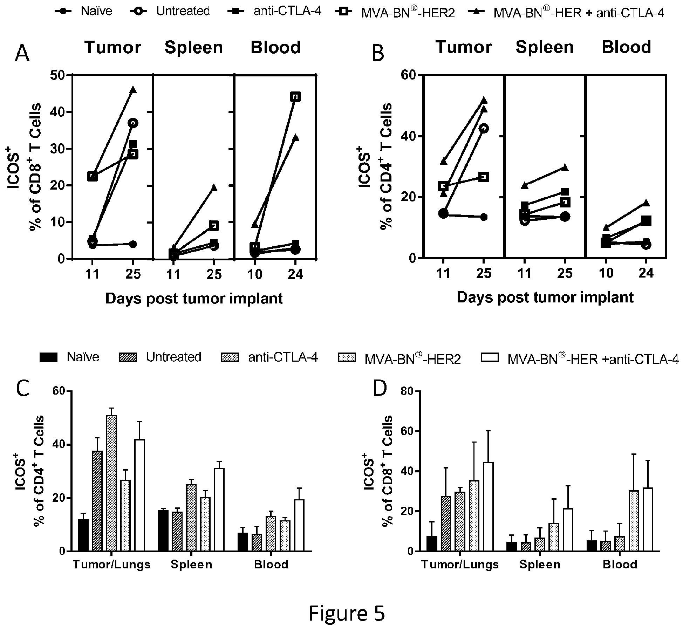

Figure 5. Shows ICOS expression increased on T cells from the tumor/lungs and in the periphery in mice treated with MVA-BN-HER2, and in the tumor/lungs of mice with high tumor burden. Mice with CT26-HER-2 tumors in the lung were treated with MVA-BN-HER2 on days 4 and 18, and anti-CTLA-4 on days 3 and 17. A) ICOS expression on CD4+ T cells at day 11 and 25. B) ICOS expression on CD8+ T cells at day 11 and 25. C) Average ICOS expression on CD4+ T cells at day 25 from three independent experiments with 3-4 mice per group. D) Average ICOS expression on CD8+ T cells at day 25 from three independent experiments with 3-4 mice per group.

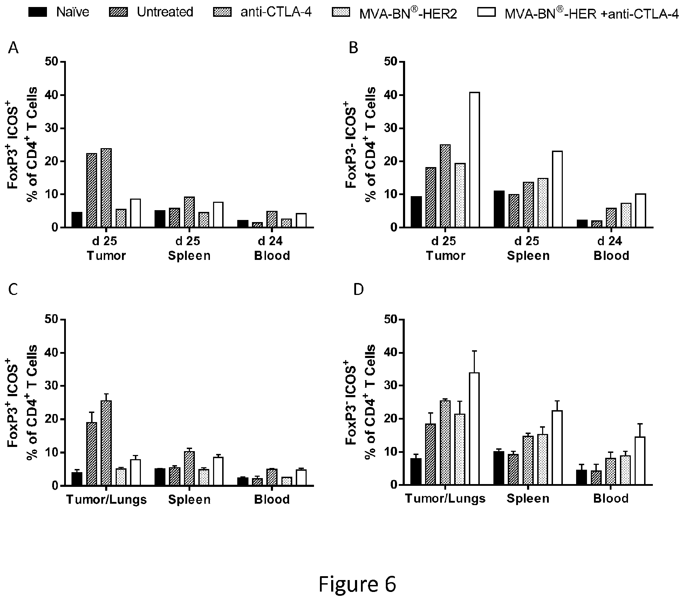

Figure 6. Shows ICOS+ CD4+ T Cells are approximately equal proportions of FoxP3+ in tumor bearing mice, but are primarily FoxP3- in mice responding to treatment. Mice with CT26-HER-2 tumors in the lung were treated with MVA-BN-HER2 on days 4 and 18, and anti-CTLA-4 on days 3 and 17 and FACS analysis performed on day 24 or 25 after tumor implant. A) ICOS expression on FoxP3+ Tregs. B) ICOS expression on FoxP3- CD4 T cells. C) Average ICOS expression on FoxP3+ Tregs from three independent experiments with 3-4 mice per group. D) ICOS expression on FoxP3- CD4 T from three independent experiments with 3-4 mice per group.

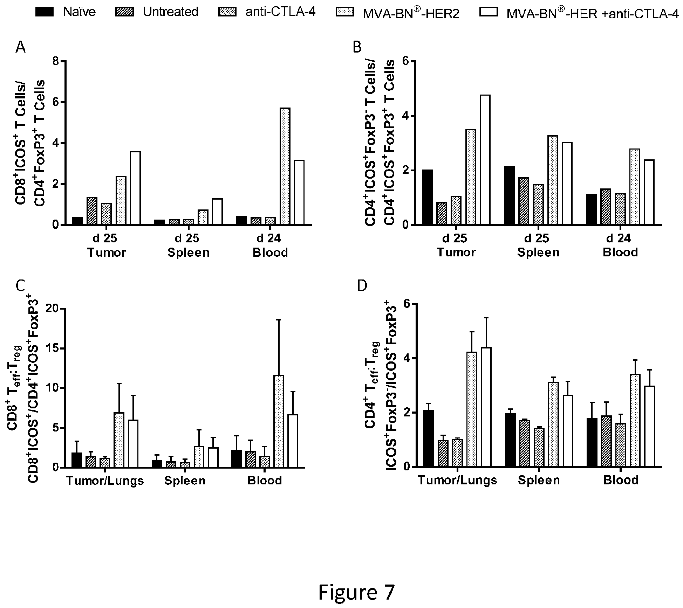

Figure 7. Shows MVA-BN-HER2 and combination treatment with anti-CTLA-4 increased the effector to regulatory T cell ratio. Mice with CT26-HER-2 tumors in the lung were treated with MVA-BN-HER2 on days 4 and 18, and anti-CTLA-4 on days 3 and 17 and FACS analysis performed on day 24 or 25 after tumor implant. A) CD8 Teff:Treg ratio (CD8+ICOS+FoxP3-/CD4+ICOS+FoxP3+) and B) CD4 Teff:Treg ratio (CD4+ICOS+FoxP3-/CD4+ICOS+FoxP3 from a single experiment with 3-4 mice per group. C) Average CD8 Teff:Treg ratio and D) Average CD4 Teff:Treg ratio from three independent experiments with 3-4 mice per group.

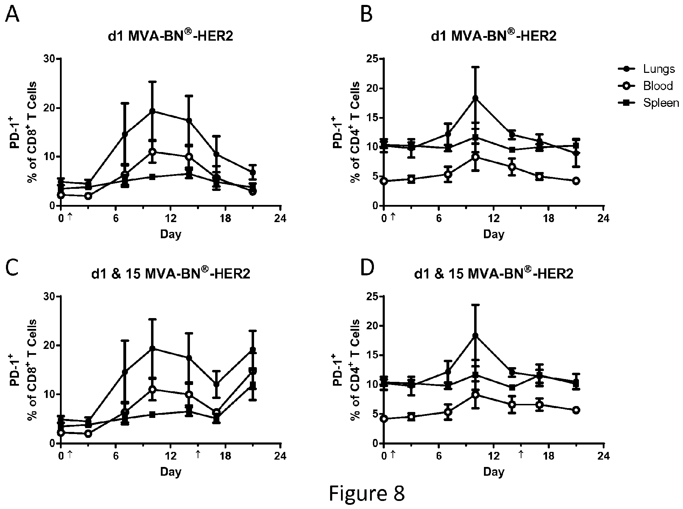

Figure 8. Shows PD-1 expression in the lungs, spleen, and blood on T cells after treatment with MVA-BN-HER2. Naive (tumor-free) mice were treated on day 1 (A and B) or days 1 and 15 (C and D) with MVA-BN-HER2. PD-1 expression on CD8+ T cells (A and C) and CD4+ T cells (B and D). Data shown as mean ± SEM, three mice per group at each time point.

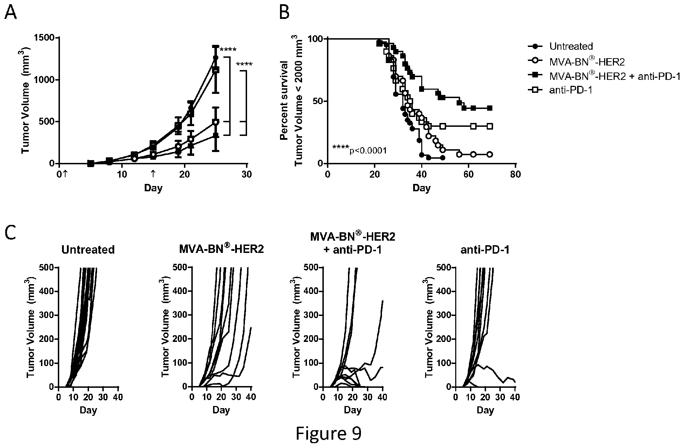

Figure 9. Shows MVA-BN-HER2 treatment and anti-PD-1 slow tumor growth and increase survival. Mice were implanted with solid CT26-HER-2 solid tumors and treated on days 1 and 15 with MVA-BN-HER2 and anti-PD-1. A) Average tumor volume in mice. B) Percent survival in mice based on tumor volume <2000 mm³. C) Individual tumor growth in mice.

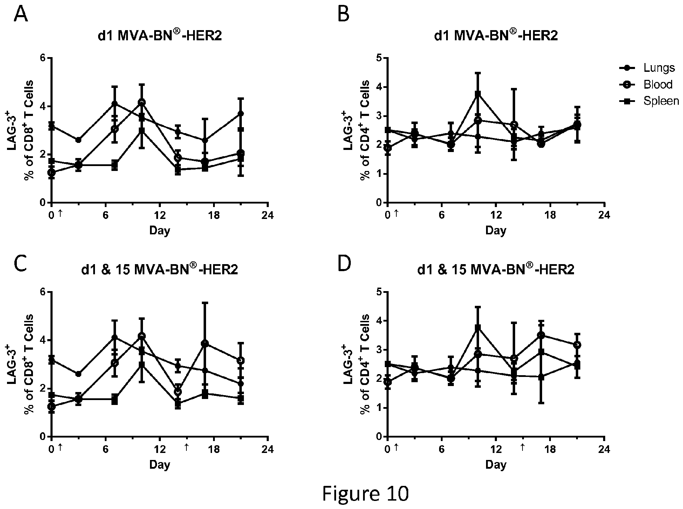

Figure 10. Shows LAG-3 expression in the lungs, spleen, and blood on T cells after treatment with MVA-BN-HER2. Naive (tumor-free) mice were treated on day 1 (A and B) or days 1 and 15 (C and D) with MVA-BN-HER2. LAG-3 expression on CD8+ T cells (A and C) and CD4+ T cells (B and D). Data shown as mean ± SEM, three mice per group at each time point.

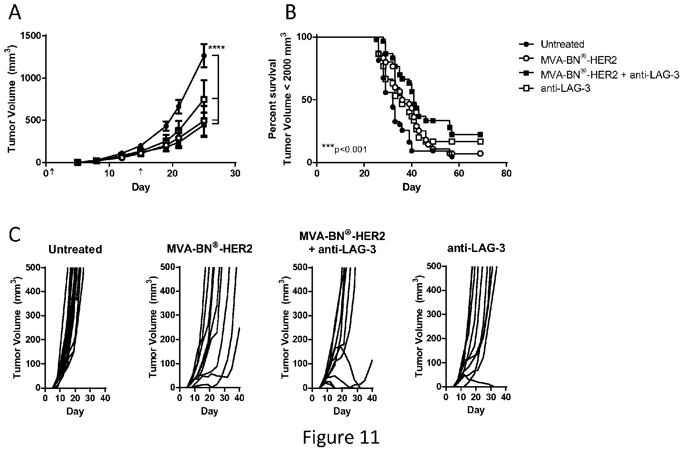

Figure 11. Shows MVA-BN-HER2 and anti-LAG-3 slow tumor growth and increase survival. Mice were implanted with solid CT26-HER-2 tumors on day 1 and treated on days 1 and 15 with MVA-BN-HER2 and anti-LAG-3. A) Average tumor volume in mice. B) Percent Survival in mice based on tumor volume <2000 mm³. C) Individual tumor growth in mice.

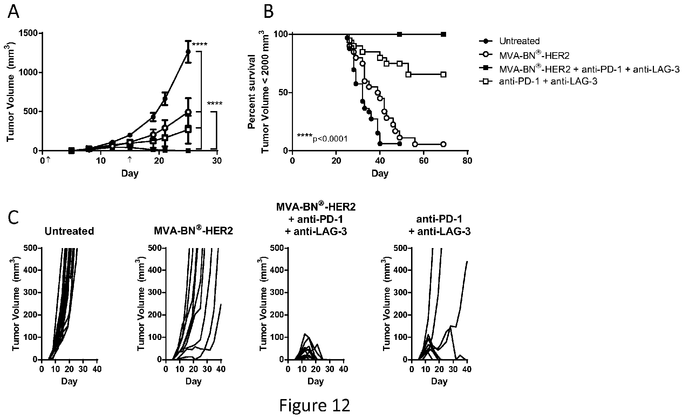

Figure 12. Shows MVA-BN-HER2, anti-PD-1, and anti-LAG-3 treatment leads to complete tumor regression in mice. Mice were implanted with solid CT26-HER-2 tumors on day 1 and treated on days 1 and 15 with MVA-BN-HER2, anti-PD-1, and anti-LAG-3. A) Average tumor volume in mice. B) Percent Survival in mice based on tumor volume <2000 mm³. C) Individual tumor growth in mice.

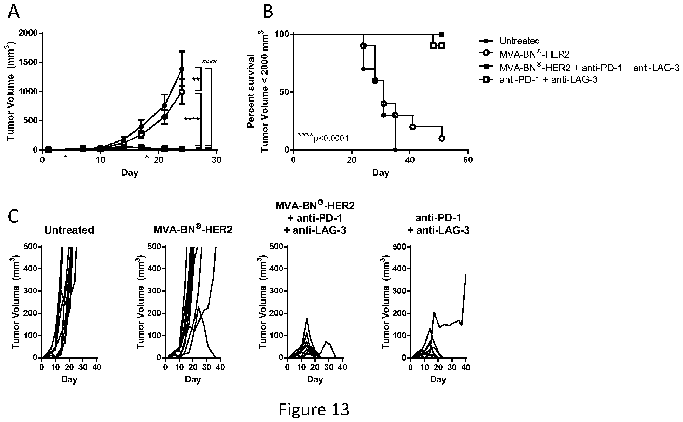

Figure 13. Shows MVA-BN-HER2, anti-PD-1, and anti-LAG-3 treatment leads to tumor regression in mice. Mice were implanted with solid CT26-HER-2 tumors on day 1 and treated on days 4 and 18 with MVA-BN-HER2, anti-PD-1, and anti-LAG-3. (As compared to Figure 12, treatment was delayed to days 4 and 18 (Figure 12, on days 1 and 15). (A) Average tumor volume in mice. B) Percent Survival in mice based on tumor volume <2000 mm³. C) Individual tumor growth in mice.

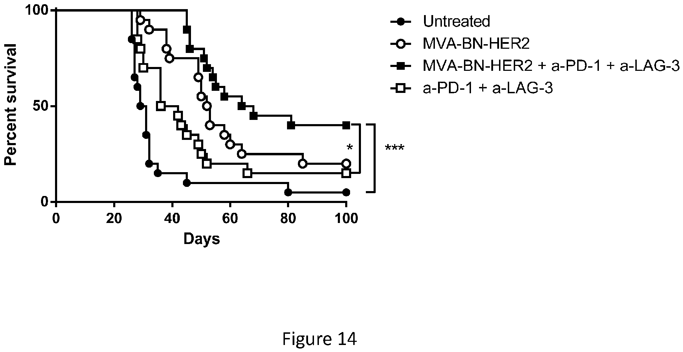

Figure 14. Shows MVA-BN-HER2 alone and in combination with anti-PD-1 and anti-LAG-3 increase survival in an experimental lung metastasis model. Mice with CT26-HER-2 tumors in the lung were treated on day 4 and 18 with MVA-BN-HER2, anti-PD-1, and anti-LAG-3.

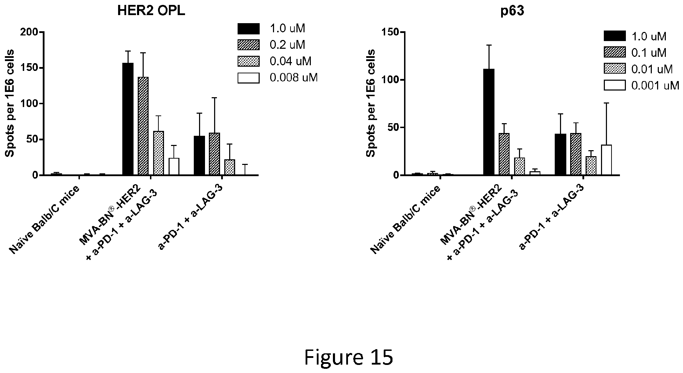

Figure 15. Shows the HER2 specific T-cell responses of mice treated with MVA-BN-HER2, anti-PD-1, and anti-LAG-3 antibodies were higher with triple combination therapy. Mice were implanted with solid CT26-HER-2 tumors on day 1 and treated on days 4 and 18 with MVA-BN-HER2, anti-PD-1, and anti-LAG-3 and IFN-γ was measured by ELISPOT four weeks after the last treatment. Splenocytes from tumor free mice were re-stimulated with HER-2 ECD overlapping peptide library (A, 166 overlapping 15mers) or the K^{d} binding HER-2 peptide p63 (B).

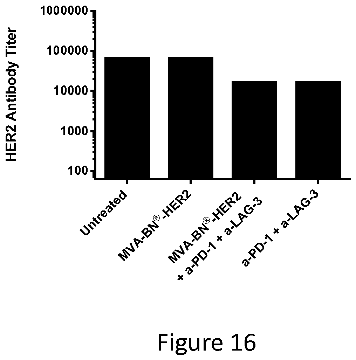

Figure 16. Shows the growing CT26-HER-2 tumor induced HER2 specific antibodies that are similar among all treatment groups. Mice were implanted with solid CT26-HER-2 tumors on day 1 and treated on days 4 and 18 with MVA-BN-HER2, anti-PD-1, and anti-LAG-3. Serum was collected on day 25 from mice and HER-2 titers measured by ELISA.

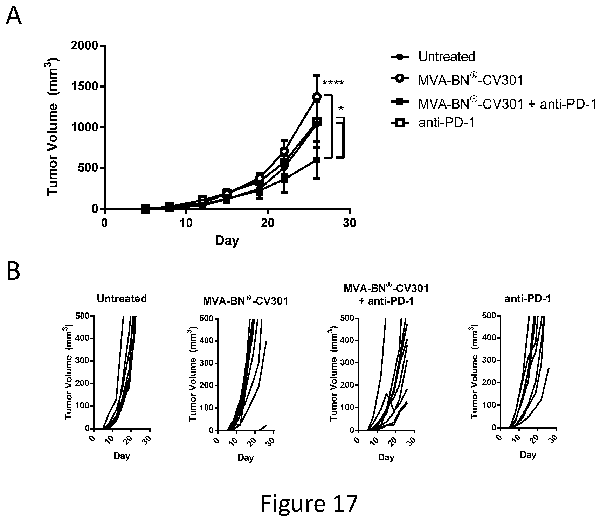

Figure 17. Shows MVA-BN-CV301 and anti-PD-1 slow tumor growth. Mice were implanted with MC38-CEA solid tumors and treated on days 1 and 15 with MVA-BN-CV301 and anti-PD-1. A) Average tumor volume in mice. B) Individual tumor growth in mice.

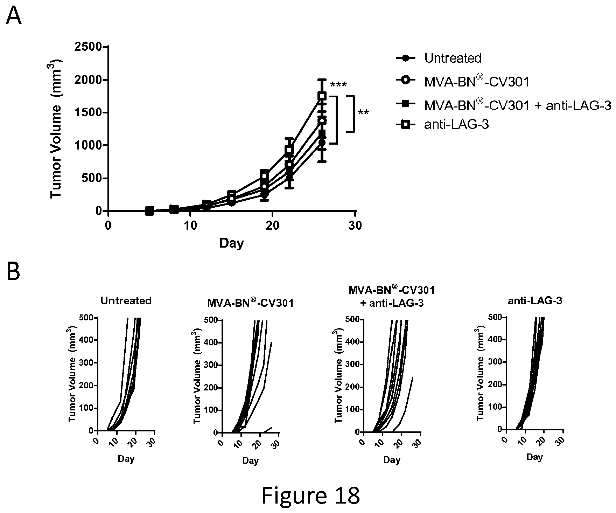

Figure 18. Shows tumor volume after administration of MVA-BN-CV301 and anti-LAG-3. Mice were implanted with MC38-CEA solid tumors and treated on days 1 and 15 with MVA-BN-CV301 and anti-LAG-3. A) Average tumor volume in mice. B) Individual tumor growth in mice.

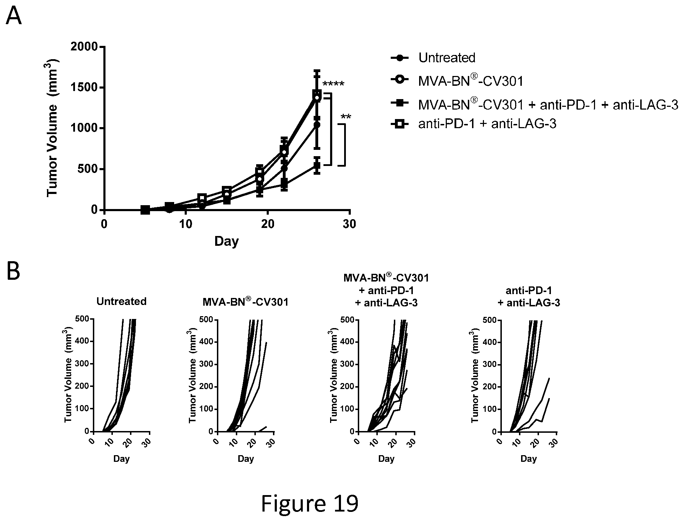

Figure 19. Shows tumor volume after administration MVA-BN-CV301 in combination with anti-PD-1 and anti-LAG-3. Mice were implanted with MC38-CEA solid tumors and treated on days 1 and 15 with MVA-BN-CV301, anti-PD-1, and anti-LAG-3. A) Average tumor volume in mice. B) Individual tumor growth in mice.

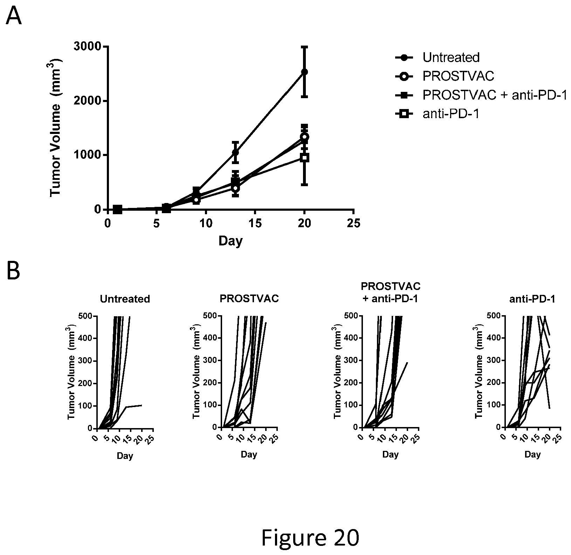

Figure 20. Shows tumor volume after administration of PROSTVAC and anti-PD-1. Mice were implanted with E6 (RM11-PSA) solid tumors and treated on day 1 with PROSTVAC-V, and days 8 and 15 with PROSTVAC-F. Anti-PD-1 was given on days 1 and 15. A) Average tumor volume in mice. B) Individual tumor growth in mice.

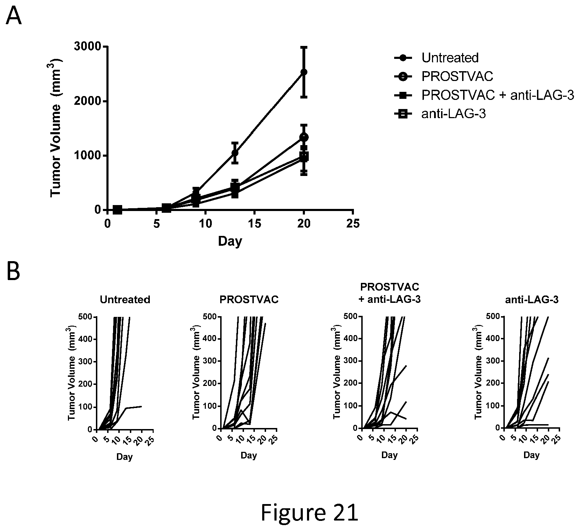

Figure 21. Shows tumor volume after administration of PROSTVAC and anti-LAG-3. Mice were implanted with E6 (RM11-PSA) solid tumors and treated on day 1 with PROSTVAC-V and days 8 and 15 with PROSTVAC-F. Anti-LAG-3 was given on days 1 and 15. A) Average tumor volume in mice. B) Individual tumor growth in mice.

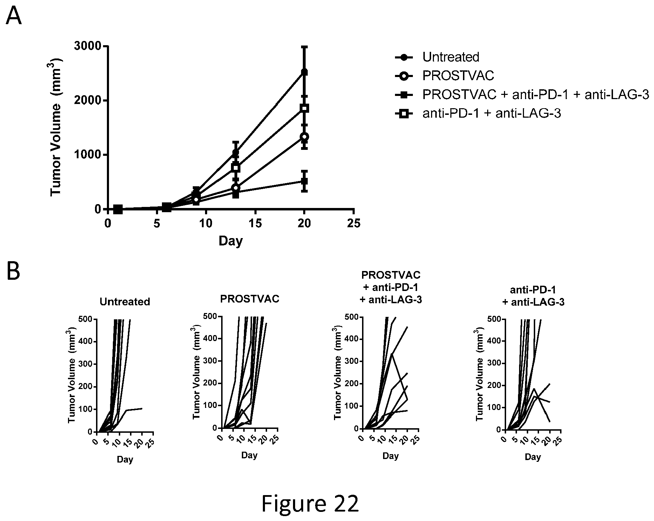

Figure 22. Shows tumor volume after administration of PROSTVAC, anti-PD-1, and anti-LAG-3. Mice were implanted with E6 (RM11-PSA) solid tumors and treated on day 1 with PROSTVAC-V and days 8 and 15 with PROSTVAC-F. Anti-PD-1 and anti-LAG-3 were given on days 1 and 15. A) Average tumor volume in mice. B) Individual tumor growth in mice.

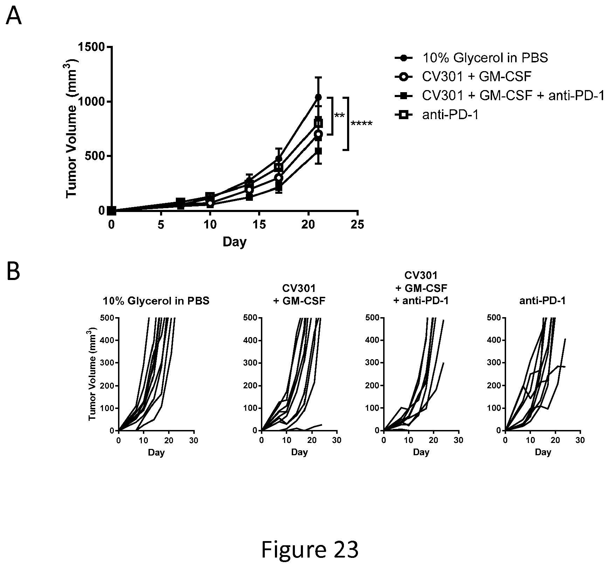

Figure 23. Shows tumor volume after administration of CV301 and anti-PD-1. CEA transgenic mice were implanted with MC38-CEA solid tumors and treated with CV301-V on day 4 CV301-F on days 11 and 18. Fowlpox GM-CSF (admixed with CV301-V/F) and anti-PD-1 were given on days 4, 11, and 18. A) Average tumor volume in mice. B) Individual tumor growth in mice.

### BRIEF DESCRIPTION OF THE SEQUENCES

SEQ ID NO: 1 is the nucleotide sequence of a construct encoding a HER2 protein including two T_{H}-cell epitopes derived from tetanus toxin.
SEQ ID NO: 2 is an amino acid sequence of the modified HER2 protein encoded by the nucleotide sequence of SEQ ID NO: 1.

### DETAILED DESCRIPTION OF THE INVENTION

A number of current clinical trials involve therapies employing vaccinia, Modified Vaccinia Ankara (MVA), and fowlpox-based vectors that were engineered to express one or more tumor-associated antigens (TAA). These vectors are used alone or in prime-boost strategies to generate an active immune response against a variety of cancers. PROSTVAC® employs a heterologous prime-boost strategy using vaccinia and fowlpox expressing PSA and TRICOM™ and is currently in a global Phase III clinical trial (PROSPECT) for castration-resistant metastatic prostate cancer.

MVA-BN-HER2 (Mandl et al, 2012) is in Phase I clinical trials for the treatment of HER-2⁺-breast cancer. This recombinant vector is derived from the highly attenuated Modified Vaccinia Ankara (MVA) virus stock known as MVA-BN. It expresses a modified form of HER-2 (designated HER2) consisting of the extracellular domain of HER-2 that has been engineered to include two universal T cell epitopes from tetanus toxin (TTp2 and TTp30) to facilitate the induction of effective immune responses against HER-2.

To further enhance the anti-tumor efficacy of the poxvirus-based immunotherapy, MVA-BN-HER2 was combined with a monoclonal antibody that blocks the activity of CTLA-4, an immune checkpoint protein that down-regulates T cell activation. In the CT26-HER-2 experimental lung metastasis model, the median survival time increased from 30 days in untreated mice to 49.5 days with MVA-BN-HER2 treatment while anti-CTLA-4 treatment by itself showed little survival benefit (median survival 35 days). In contrast, MVA-BN-HER2 in combination with anti-CTLA-4 significantly increased the survival to greater than 100 days (p<0.0001) in more than 50% of the mice. At 100 days, the lungs of the surviving mice were examined and there were no visible tumors. In separate experiments, phenotypic analysis was performed. MVA-BN-HER2 therapy led to a dramatic increase in the inducible co-stimulatory molecule (ICOS) on CD8⁺ T cells in the lungs of naive mice (no tumors). In tumor bearing mice at day 25, there was an increase in the number of regulatory T cells (CD4⁺FoxP3⁺) in the lungs of untreated and anti-CTLA-4 treated mice which correlated with increased pulmonary tumor burden. The regulatory T cells were positive for ICOS. Mice treated with MVA-BN-HER2 had an increase in ICOS⁺ CD4⁺ T-cells that were FoxP3 negative.

MVA-BN-HER2 was tested in combination with various agonist and antagonist antibodies directed against PD-1, LAG-3, and ICOS in various tumor models. Combinations were found to enhance the effects of MVA-BN-HER2.

Additionally, various antagonist antibodies directed against PD-1 and LAG-3 were tested in combination with heterologous-prime boost dosing regimens utilizing a recombinant orthopoxvirus and a recombinant avipoxvirus. For example, PROSTVAC®, which includes a Vaccinia virus and a Fowlpox virus, each expressing PSA and TRICOM, was tested in combination with the various antagonist antibodies. CV301 (also known as PANVAC), which includes a Vaccinia virus and a Fowlpox Virus, each expressing CEA, MUC-1, and TRICOM, was also tested in combination with the various antagonist antibodies. The effectiveness of the heterologous prime-boost dosing regimens increased when administered in combination with the various antagonist antibodies.

Various antagonist antibodies directed against PD-1 and LAG-3 were additionally tested in the homologous prime-boost regimen of MVA-CV301. The effectiveness of MVA-CV301, which includes a prime and boosting dosing of an MVA virus expressing CEA, MUC-1, and TRICOM, was found to be improved when tested in combination with the antibodies directed against PD-1 and/or LAG-3.

### Orthopoxvirus and/or Avipoxvirus encoding a polypeptide comprising a tumor antigen

In various aspects, the present disclosure includes a recombinant orthopoxvirus and/or a recombinant avipoxvirus each encoding and/or expressing a tumor antigen. In one or more preferred aspects, the orthopoxvirus and the avipoxvirus are a vaccinia virus and a fowlpox virus, respectively.

The term "avipoxvirus" refers to any avipoxvirus, such as Fowlpoxvirus, Canarypoxvirus, Uncopoxvirus, Mynahpoxvirus, Pigeonpoxvirus, Psittacinepoxvirus, Quailpoxvirus, Peacockpoxvirus, Penguinpoxvirus, Sparrowpoxvirus, Starlingpoxvirus and Turkeypoxvirus. Preferred avipoxviruses are Canarypoxvirus and Fowlpoxvirus.

An example of a canarypox virus is strain Rentschler. A plaque purified Canarypox strain termed ALVAC (U.S. Pat. No. 5,766,598) was deposited under the terms of the Budapest treaty with the American Type Culture Collection (ATCC), accession number VR-2547. Another Canarypox strain is the commercial canarypox vaccine strain designated LF2 CEP 524 24 10 75, available from Institute Merieux, Inc.

Examples of a Fowlpox virus include, but are not limited to, strains FP-1, FP-5, TROVAC (U.S. Pat. No. 5,766,598), and POXVAC-TC (U.S. Patent 7,410,644). FP-1 is a Duvette strain modified to be used as a vaccine in one-day old chickens. The strain is a commercial fowlpox virus vaccine strain designated O DCEP 25/CEP67/2309 October 1980 and is available from Institute Merieux, Inc. FP-5 is a commercial fowlpox virus vaccine strain of chicken embryo origin available from American Scientific Laboratories (Division of Schering Corp.) Madison, Wis., United States Veterinary License No. 165, serial No. 30321.

In one or more embodiments, the recombinant orthopoxvirus is preferably selected from a vaccinia virus, a modified vaccinia Ankara (MVA) virus, and MVA-BN.

Examples of vaccinia virus strains include, but are not limited to, the strains Temple of Heaven, Copenhagen, Paris, Budapest, Dairen, Gam, MRIVP, Per, Tashkent, TBK, Tom, Bern, Patwadangar, BIEM, B-15, Lister, EM-63, New York City Board of Health, Elstree, Ikeda and WR. A preferred vaccinia virus (VV) strain is the Wyeth (DRYVAX) strain (U.S. Patent 7,410,644).

Another preferred VV strain is a modified vaccinia Ankara (MVA) virus (Sutter, G. et al. [1994], Vaccine 12: 1032-40). Examples of MVA virus strains that are useful in the practice of the present invention and that have been deposited in compliance with the requirements of the Budapest Treaty include, but are not limited to, strains MVA 572, deposited at the European Collection of Animal Cell Cultures (ECACC), Vaccine Research and Production Laboratory, Public Health Laboratory Service, Centre for Applied Microbiology and Research, Porton Down, Salisbury, Wiltshire SP4 0JG, United Kingdom, with the deposition number ECACC 94012707 on January 27, 1994, and MVA 575, deposited under ECACC 00120707 on December 7, 2000. MVA-BN, deposited on Aug. 30, 2000 at the European Collection of Cell Cultures (ECACC) under number V00083008, and its derivatives, are additional exemplary strains.

Although MVA-BN is preferred for its higher safety (less replication competent), all MVAs are suitable for this invention. According to an embodiment of the present invention, the MVA strain is MVA-BN and its derivatives. A definition of MVA-BN and its derivatives is given in PCT/EP01/13628..

In certain embodiments, the MVA is MVA-BN, deposited on Aug. 30, 2000, at the European Collection of Cell Cultures (ECACC) under number V00083008, and described in International PCT publication WO2002042480 (see also e.g. U.S. Pat. Nos. 6,761,893 and 6,913,752). As described in those patent publications, MVA-BN does not reproductively replicate in cell lines 293, 143B, HeLa and HaCat. In particular, MVA-BN exhibits an amplification ratio of 0.05 to 0.2 in the human embryo kidney cell line 293. In the human bone osteosarcoma cell line 143B, MVA-BN exhibits an amplification ratio of 0.0 to 0.6. MVA-BN exhibits an amplification ratio of 0.04 to 0.8 in the human cervix adenocarcinoma cell line HeLa, and 0.02 to 0.8 in the human keratinocyte cell line HaCat. MVA-BN has an amplification ratio of 0.01 to 0.06 in African green monkey kidney cells (CV1: ATCC No. CCL-70).

The amplification ratio of MVA-BN is above 1 in chicken embryo fibroblasts (CEF: primary cultures) as described in PCT publication WO2002042480 (see also e.g. U.S. Pat. Nos. 6,761,893 and 6,913,752). The virus can be easily propagated and amplified in CEF primary cultures with a ratio above 500.

In certain embodiments, a recombinant MVA is a derivative of MVA-BN. Such "derivatives" include viruses exhibiting essentially the same replication characteristics as the deposited strain (ECACC No. V00083008), but exhibiting differences in one or more parts of its genome. Viruses having the same "replication characteristics" as the deposited virus are viruses that replicate with similar amplification ratios as the deposited strain in CEF cells and the cell lines, HeLa, HaCat and 143B; and that show similar replication characteristics in vivo, as determined, for example, in the AGR129 transgenic mouse model.

In certain embodiments, the orthopoxvirus is a recombinant vaccinia virus that contains additional nucleotide sequences that are heterologous to the orthopoxvirus. In certain such embodiments, the heterologous sequences code for epitopes that induce a response by the immune system. Thus, in certain embodiments, the recombinant orthopoxvirus is used to vaccinate against the proteins or agents comprising the epitope.

In certain embodiments, the orthopoxvirus and avipoxvirus in accordance with the present disclosure comprise at least one tumor-associated antigen. In a preferred embodiment, the tumor-associated antigen includes, but is not limited to a HER-2, PSA, PAP, CEA, or MUC-1 antigen alone or in combinations (e.g., CEA and MUC-1 or PAP and PSA),.

In further embodiments, the tumor-associated antigen is modified to include one or more foreign T_{H} epitopes. Such a cancer immunotherapeutic agent is described herein in a non-limiting example and is referred to as "MVA-BN-mHER2." As described herein, such cancer immunotherapeutic agents, including, but not limited to MVA-BN-mHER2, are useful for the treatment of cancer. The invention allows for the use of such agents in prime/boost vaccination regimens of humans and other mammals, including immunocompromised patients; and inducing both humoral and cellular immune responses, such as inducing a Th1 immune response in a pre-existing Th2 environment.

In certain embodiments, the tumor associated antigen is embodied in a heterologous nucleic acid sequence that is inserted into a non-essential region of the virus genome. In certain of those embodiments, the heterologous nucleic acid sequence is inserted at a naturally occurring deletion site of the MVA genome as described in PCT/EP96/02926. Methods for inserting heterologous sequences into the poxviral genome are known to a person skilled in the art.

In certain embodiments, pharmaceutical compositions comprise one or more pharmaceutically acceptable and/or approved carriers, additives, antibiotics, preservatives, adjuvants, diluents and/or stabilizers. Such additives include, for example, but not limited to, water, saline, glycerol, ethanol, wetting or emulsifying agents, and pH buffering substances. Exemplary carriers are typically large, slowly metabolized molecules such as proteins, polysaccharides, polylactic acids, polyglycolic acids, polymeric amino acids, amino acid copolymers, lipid aggregates, or the like.

For the preparation of vaccines, the orthopoxvirus can be converted into a physiologically acceptable form. In certain embodiments, such preparation is based on experience in the preparation of poxvirus vaccines used for vaccination against smallpox, as described, for example, in Stickl, H. et al., Dtsch. med. Wschr. 99, 2386-2392 (1974).

An exemplary preparation follows. Purified virus is stored at -80°C with a titer of 5 x 10⁸ TCID₅₀/ml formulated in 10 mM Tris, 140 mM NaCl, pH 7.4. For the preparation of vaccine shots, e.g., 10²-10⁸ particles of the virus can be lyophilized in phosphate-buffered saline (PBS) in the presence of 2% peptone and 1% human albumin in an ampoule, preferably a glass ampoule. Alternatively, the vaccine shots can be prepared by stepwise, freeze-drying of the virus in a formulation. In certain embodiments, the formulation contains additional additives such as mannitol, dextran, sugar, glycine, lactose, polyvinylpyrrolidone, or other additives, such as, including, but not limited to, antioxidants or inert gas, stabilizers or recombinant proteins (e.g. human serum albumin) suitable for *in vivo* administration. The ampoule is then sealed and can be stored at a suitable temperature, for example, between 4°C and room temperature for several months. However, as long as no need exists, the ampoule is stored preferably at temperatures below -20°C.

In various embodiments involving vaccination or therapy, the lyophilisate is dissolved in 0.1 to 0.5 ml of an aqueous solution, preferably physiological saline or Tris buffer, and administered either systemically or locally, i.e., by parenteral, subcutaneous, intravenous, intramuscular, intranasal, intradermal, or any other path of administration known to a skilled practitioner. Optimization of the mode of administration, dose, and number of administrations is within the skill and knowledge of one skilled in the art.

In certain embodiments, attenuated vaccinia virus strains are useful to induce immune responses in immune-compromised animals, e.g., monkeys (CD4<400/µl of blood) infected with SIV, or immune-compromised humans. The term "immune-compromised" describes the status of the immune system of an individual that exhibits only incomplete immune responses or has a reduced efficiency in the defense against infectious agents.

### Certain Exemplary Tumor-Associated Antigens

In certain embodiments, an immune response is produced in a subject against a cell-associated polypeptide antigen. In certain such embodiments, a cell-associated polypeptide antigen is a tumor-associated antigen.

The term "polypeptide" refers to a polymer of two or more amino acids joined to each other by peptide bonds or modified peptide bonds. The amino acids may be naturally occurring as well as non-naturally occurring, or a chemical analogue of a naturally occurring amino acid. The term also refers to proteins, i.e. functional biomolecules comprising at least one polypeptide; when comprising at least two polypeptides, these may form complexes, be covalently linked, or may be non-covalently linked. The polypeptide(s) in a protein can be glycosylated and/or lipidated and/or comprise prosthetic groups.

As described herein, preferably, the tumor-associated antigen is HER-2, PSA, PAP, CEA, or MUC-1, alone or in combinations (e.g., CEA and MUC-1 or PAP and PSA).

Numerous tumor-associated antigens are known in the art. Exemplary tumor-associated antigens include, but are not limited to, 5 alpha reductase, alpha-fetoprotein, AM-1, APC, April, BAGE, beta-catenin, Bcl12, bcr-abl, CA-125, CASP-8/FLICE, Cathepsins, CD19, CD20, CD21, CD23, CD22, CD33 CD35, CD44, CD45, CD46, CD5, CD52, CD55, CD59, CDC27, CDK4, CEA, c-myc, Cox-2, DCC, DcR3, E6/E7, CGFR, EMBP, Dna78, farnesyl transferase, FGF8b, FGF8a, FLK-1/KDR, folic acid receptor, G250, GAGE-family, gastrin 17, gastrin-releasing hormone, GD2/GD3/GM2, GnRH, GnTV, GP1, gp100/Pmel17, gp-100-in4, gp15, gp75/TRP-1, hCG, heparanse, Her2/neu, HMTV, Hsp70, hTERT, IGFR1, IL-13R, iNOS, Ki67, KIAA0205, K-ras, H-ras, N-ras, KSA, LKLR-FUT, MAGE-family, mammaglobin, MAP17, melan-A/MART-1, mesothelin, MIC A/B, MT-MMPs, mucin, NY-ESO-1, osteonectin, p15, P170/MDR1, p53, p97/melanotransferrin, PAI-1, PDGF, uPA, PRAME, probasin, progenipoientin, PSA, PSM, RAGE-1, Rb, RCAS1, SART-1, SSX-family, STAT3, STn, TAG-72, TGF-alpha, TGF-beta, Thymosin-beta-15, TNF-alpha, TRP-1, TRP-2, tyrosinase, VEGF, ZAG, p16INK4, and glutathione-S-transferase.

A preferred PSA antigen comprises the amino acid change of isoleucine to leucine at position 155, as described in U.S. Patent 7,247,615.

One or more preferred CEA antigens include, but are not limited to, CEA antigens described in US Patent 7,723,096 and PCT application Nos. PCT/US2004/037810 and PCT/US2004/038643.

One or more preferred MUC-1 antigens include, but are not limited to, MUC-1 antigens described in US Patent 7,118,738 and PCT application Nos. PCT/US2013/020058, PCT/US2004/037810, and PCT/US2004/038643.

Another exemplary tumor-associated antigen is HER-2. HER-2 is a member of the epidermal growth factor receptor family (c-erbB) which consists of four different receptors to date: c-erbB-1 (EGFr), c-erbB-2 (HER-2, c-Neu), c-erbB-3 and c-erbB-4 (Salomon et al, 1995). C-erbB-3 and c-erbB-4 are less well characterized than EGFr and HER-2. HER-2 is an integral membrane glycoprotein. The mature protein has a molecular weight of 185 kD with structural features that closely resemble the EGFr receptor (Prigent et al, 1992). EGFr is also an integral membrane receptor consisting of one subunit. It has an apparent molecular weight of 170 kD and consists of a surface ligand-binding domain of 621 amino acids, a single hydrophobic transmembrane domain of 23 amino acids, and a highly conserved cytoplasmic tyrosine kinase domain of 542 amino acids. The protein is N-glycosylated (Prigent et al, 1994).

All proteins in this family are tyrosine kinases. Interaction with the ligand leads to receptor dimerization, which increases the catalytic action of the tyrosine kinase (Bernard. 1995, Chantry 1995). The proteins within the family are able to homo- and heterodimerise, which is important for their activity. The EGFr conveys growth promoting effects and stimulates uptake of glucose and amino acids by cells (Prigent et al 1992). HER-2 also conveys growth promoting signals.

The epidermal growth factor receptor is expressed on normal tissues in low amounts, but it is overexpressed in many types of cancers. EGFr is overexpressed in breast cancers (Earp et al, 1993, Eppenberger 1994), gliomas (Schlegel et al, 1994), gastric cancer (Tkunaga et al, 1995), cutaneous squamous carcinoma (Fujii 1995), ovarian cancer (van Dam et al, 1994) and others. HER-2 is also expressed on few normal human tissues in low amount, most characteristically on secretory epithelia. Over-expression of HER-2 occurs in about 30% of breast, gastric, pancreatic, bladder and ovarian cancers.

The expression of these receptors varies depending on the degree of differentiation of the tumors and the cancer type, e.g., in breast cancer, primary tumors overexpress both receptors; whereas in gastric cancer, the overexpression occurs at a later stage in metastatic tumours (Salomon et al, 1995). The number of overexpressed receptors on carcinoma cells is greater than 10⁶/cell for several head and neck cancers, vulva, breast and ovarian cancer lines isolated from patients (Dean et al, 1994).

There are several reasons why the EGFr family of receptors constitutes suitable targets for tumor immunotherapy. First, they are overexpressed in many types of cancers, which should direct the immune response towards the tumor. Second, the tumors often express or overexpress the ligands for this family of receptors and some are hypersensitive to the proliferative effects mediated by the ligands. Third, patients with tumors that overexpress growth factor receptors often have a poor prognosis. The overexpression has been closely linked with poor prognosis especially in breast cancer, lung cancer, and bladder cancer and can be associated with invasive/metastatic phenotypes, which are rather insensitive to conventional therapies (Eccles et al, 1994).

It is contemplated that the nucleic acids encoding the tumor antigen (or tumor associated antigen) can be operatively linked to expression control sequences. An expression control sequence operatively linked to a coding sequence is joined such that expression of the coding sequence is achieved under conditions compatible with the expression control sequences. The expression control sequences include, but are not limited to, appropriate promoters, enhancers, transcription terminators, a start codon at the beginning a protein-encoding open reading frame, splicing signals for introns, and in-frame stop codons. Suitable promoters include, but are not limited to, the SV40 early promoter, an RSV promoter, the retrovirus LTR, the adenovirus major late promoter, the human CMV immediate early I promoter, and various poxvirus promoters including, but not limited to the following vaccinia virus or MVA-derived promoters: the ATI promoter, the 30K promoter, the I3 promoter, the PrS promoter, the Pr7.5K, the 40K promoter, the PrSynIIm promoter, the PrLE1 promoter, and the PrSSL promoter (as described in PCT Application PCT/EP2009/008459).

Additional expression control sequences include, but are not limited to, leader sequences, termination codons, polyadenylation signals and any other sequences necessary for the appropriate transcription and subsequent translation of the nucleic acid sequence encoding the desired recombinant protein (e.g., HER-2, PSA, PAP, CEA, or MUC-1) in the desired host system. The poxvirus vector may also contain additional elements necessary for the transfer and subsequent replication of the expression vector containing the nucleic acid sequence in the desired host system. It will further be understood by one skilled in the art that such vectors are easily constructed using conventional methods (Ausubel et al., (1987) in "Current Protocols in Molecular Biology," John Wiley and Sons, New York, N.Y.) and are commercially available.

### Modified Tumor-Associated Antigens

In certain embodiments, a cell-associated polypeptide antigen is modified such that a CTL response is induced against a cell which presents epitopes derived from a polypeptide antigen on its surface, when presented in association with an MHC Class I molecule on the surface of an APC. In certain such embodiments, at least one first foreign T_{H} epitope, when presented, is associated with an MHC Class II molecule on the surface of the APC. In certain such embodiments, a cell-associated antigen is a tumor-associated antigen (TAA).

Exemplary APCs capable of presenting epitopes include dendritic cells and macrophages. Additional exemplary APCs include any pino- or phagocytizing APC, which is capable of simultaneously presenting 1) CTL epitopes bound to MHC class I molecules and 2) T_{H} epitopes bound to MHC class II molecules.

In certain embodiments, modifications to one or more of the tumor-associated antigens (TAA) presented herein, such as, but not limited to, CEA, MUC-1, PAP, PSA, HER2 are made such that, after administration to a subject, polyclonal antibodies are elicited that predominantly react with the one or more of the TAAs described herein. Such antibodies could attack and eliminate tumor cells as well as prevent metastatic cells from developing into metastases. The effector mechanism of this anti-tumor effect would be mediated via complement and antibody dependent cellular cytotoxicity. In addition, the induced antibodies could also inhibit cancer cell growth through inhibition of growth factor dependent oligo-dimerisation and internalisation of the receptors. In certain embodiments, such modified polypeptide antigens could induce CTL responses directed against known and/or predicted TAA epitopes displayed by the tumor cells.

In certain embodiments, a modified TAA polypeptide antigen comprises a CTL epitope of the cell-associated polypeptide antigen and a variation, wherein the variation comprises at least one CTL epitope of a foreign T_{H} epitope. Certain such modified TAAs can include, in one non-limiting example, one or more modified HER-2 polypeptide antigens comprising at least one CTL epitope and a variation comprising at least one CTL epitope of a foreign T_{H} epitope. Methods of producing the same are described in U.S. Patent No. 7,005,498 and U.S. Patent Pub. Nos. 2004/0141958 and 2006/0008465.

In certain embodiments, a foreign T_{H} epitope is a naturally-occurring "promiscuous" T-cell epitope. Such promiscuous T-cell epitopes are active in a large proportion of individuals of an animal species or an animal population. In certain embodiments, a vaccine comprises such promiscuous T-cell epitopes. In certain such embodiments, use of promiscuous T-cell epitopes reduces the need for a very large number of different CTL epitopes in the same vaccine. Exemplary promiscuous T-cell epitopes include, but are not limited to, epitopes from tetanus toxin, including but not limited to, the P2 and P30 epitopes (Panina-Bordignon et al., 1989), diphtheria toxin, Influenza virus hemagluttinin (HA), and P. falciparum CS antigen.

Additional promiscuous T-cell epitopes include peptides capable of binding a large proportion of HLA-DR molecules encoded by the different HLA-DR. See, e.g., WO 98/23635 (Frazer IH et al., assigned to The University of Queensland); Southwood S et. al, 1998, J. Immunol. 160: 3363 3373; Sinigaglia F et al., 1988, Nature 336: 778 780; Rammensee HG et al., 1995, Immunogenetics 41: 4 178 228; Chicz RM et al., 1993, J. Exp. Med 178: 27 47; Hammer J et al., 1993, Cell 74: 197 203; and Falk K et al., 1994, Immunogenetics 39: 230 242. The latter reference also deals with HLA-DQ and -DP ligands. All epitopes listed in these references are relevant as candidate natural epitopes as described herein, as are epitopes which share common motifs with these.

In certain other embodiments, the promiscuous T-cell epitope is an artificial T-cell epitope which is capable of binding a large proportion of haplotypes. In certain such embodiments, the artificial T-cell epitope is a pan DR epitope peptide ("PADRE") as described in WO 95/07707 and in the corresponding paper Alexander J et al., 1994, Immunity 1: 751 761. **mHER2**

Various modified HER-2 polypeptide antigens and methods for producing the same are described in U.S. Patent No. 7,005,498 and U.S. Patent Pub. Nos. 2004/0141958 and 2006/0008465. Those documents describe various modified HER-2 polypeptide antigens comprising promiscuous T-cell epitopes at different positions in the HER-2 polypeptide.

The human HER-2 sequence can be divided into a number of domains based solely on the primary structure of the protein. Those domains are as follows. The extracellular (receptor) domain extends from amino acids 1-654 and contains several subdomains as follows: Domain I (N-terminal domain of mature polypeptide) extends from amino acids 1-173; Domain II (Cysteine rich domain, 24 cysteine residues) extends from amino acids 174-323; Domain III (ligand binding domain in the homologous EGF receptor) extends from amino acids 324-483; and Domain IV (Cysteine rich domain, 20 cysteine residues) extends from amino acids 484-623. The transmembrane residues extend from amino acids 654-675. The intracellular (Kinase) domain extends from amino acids 655-1235 and contains the tyrosine kinase domain, which extends from amino acids 655-1010 (core TK domain extends from 725-992); and the C-terminal domain, which extends from amino acids 1011-1235.

Selection of sites in the amino acid sequence of HER-2 to be displaced by either the P2 or P30 human T helper epitopes is described in U.S. Patent No. 7,005,498 and U.S. Patent Pub. Nos. 2004/0141958 and 2006/0008465. To summarize, the following parameters were considered:
1. Known and predicted CTL epitopes;
2. Homology to related receptors (EGFR in particular);
3. Conservation of cysteine residues;
4. Predicted loop, α-helix and β-sheet structures;
5. Potential N-glycosylation sites;
6. Prediction of exposed and buried amino acid residues;
7. Domain organization.

The CTL epitopes appear to be clustered in domain I, domain III, the TM domain and in two or three "hot spots" in the TK domain. As described in U.S. Patent No. 7,005,498 and U.S. Patent Pub. Nos. 2004/0141958 and 2006/0008465, these should be largely conserved.

Regions with a high degree of homology with other receptors are likely to be structurally important for the "overall" tertiary structure of HER-2, and hence for antibody recognition, whereas regions with low homology possibly can be exchanged with only local alterations of the structure as the consequence.

Cysteine residues are often involved in intramolecular disulphide bridge formation and are thus involved in the tertiary structure and should not be changed. Regions predicted to form alpha-helix or beta-sheet structures should be avoided as insertion points of foreign epitopes, as these regions are thought to be involved in folding of the protein.

Potential N-glycosylation sites should be conserved if mannosylation of the protein is desired.

Regions predicted (by their hydrophobic properties) to be interior in the molecule preferably should be conserved as these could be involved in the folding. In contrast, solvent exposed regions could serve as candidate positions for insertion of the model T_{H} epitopes P2 and P30.

Finally, the domain organization of the protein should be taken into consideration because of its relevance for protein structure and function.

As described in U.S. Patent No. 7,005,498 and U.S. Patent Pub. Nos. 2004/0141958 and 2006/0008465, the focus of the strategy has been to conserve the structure of the extracellular part of HER-2 as much as possible, because this is the part of the protein which is relevant as a target for neutralizing antibodies. By contrast, the intracellular part of native membrane bound HER-2 on the surface of cancer cells is inaccessible for the humoral immune system.

Various exemplary constructs using the P2 and P30 epitopes of tetanus toxin inserted in various domains of HER-2 are provided in U.S. Patent No. 7,005,498 and U.S. Patent Pub. Nos. 2004/0141958 and 2006/0008465. One exemplary modified HER-2 polypeptide antigen, referred to as "mHER2," comprises the extracellular domains and nine amino acids of the transmembrane domain; the P2 epitope inserted in Domain II between amino acid residues 273 to 287 of the modified HER-2 polypeptide; and the P30 epitope inserted in Domain IV between amino acid residues 655 to 675 of the modified HER-2 polypeptide.

### Recombinant MVA-BN-mHER2

Recombinant MVA comprising a tumor-associated antigen, e.g., MVA-BN-mHER2, is constructed as follows. The initial virus stock is generated by recombination in cell culture using a cell type permissive for replication, e.g., CEF cells. Cells are both inoculated with an attenuated vaccinia virus, e.g., MVA-BN, and transfected with a recombination plasmid (e.g., pBN279) that encodes the tumor-associated antigen, e.g., mHER2, sequence and flanking regions of the virus genome. In one non-limiting embodiment, the plasmid pBN279 contains sequences which are also present in MVA-BN (the flanking Intergenic Region between ORF 64 and 65, IGR 64/65). The mHER2 sequence is inserted between the MVA-BN sequences to allow for recombination into the MVA-BN viral genome. In certain embodiments, the plasmid also contains a selection cassette comprising one or more selection genes to allow for selection of recombinant constructs in CEF cells. In a preferred embodiment, the recombinant MVA encodes a polypeptide comprising SEQ ID NO:2.

Simultaneous infection and transfection of cultures allows homologous recombination to occur between the viral genome and the recombination plasmid. Insert-carrying virus is then isolated, characterized, and virus stocks prepared. In certain embodiments, virus is passaged in CEF cell cultures in the absence of selection to allow for loss of the region encoding the selection genes, GPT and mRFP1.

### Antagonists of PD-1 and LAG-3

In certain embodiments, the invention encompasses antagonists of PD-1 and LAG-3. An antagonist of PD-1 and LAG-3 interferes with PD-1 and LAG-3, respectively.

Such antagonists include: antibodies which specifically bind to PD-1 or LAG-3 and inhibit PD-1 or LAG-3 biological activity; antisense nucleic acids RNAs that interfere with the expression of PD-1 or LAG-3; small interfering RNAs that interfere with the expression of PD-1, LAG-3; and small molecule inhibitors of PD-1 or LAG-3.

Candidate antagonists of PD-1 or LAG-3 can be screened for function by a variety of techniques known in the art and/or disclosed within the instant application, such as ability to interfere with inhibition by PD-1 or LAG-3 function in an in vitro or mouse model.

### Agonists of ICOS

The disclosure further encompasses agonists of ICOS. An agonist of ICOS activates ICOS. The agonist may be ICOS-L, an ICOS natural ligand. The agonist can be a mutated form of ICOS-L that retains binding and activation properties. Mutated forms of ICOS-L can be screened for activity in stimulating ICOS in vitro.

Preferably, the agonist of ICOS is an antibody.

### Antibodies

The antagonist of PD-1 or LAG-3 or the agonist of ICOS is an antibody. Antibodies can be synthetic, monoclonal, or polyclonal and can be made by techniques well known in the art. Such antibodies specifically bind to PD-1, LAG-3, or ICOS via the antigen-binding sites of the antibody (as opposed to non-specific binding). PD-1, LAG-3, or ICOS polypeptides, fragments, variants, fusion proteins, etc., can be employed as immunogens in producing antibodies immunoreactive therewith. More specifically, the polypeptides, fragment, variants, fusion proteins, etc. contain antigenic determinants or epitopes that elicit the formation of antibodies.

These antigenic determinants or epitopes can be either linear or conformational (discontinuous). Linear epitopes are composed of a single section of amino acids of the polypeptide, while conformational or discontinuous epitopes are composed of amino acids sections from different regions of the polypeptide chain that are brought into close proximity upon protein folding (C. A. Janeway, Jr. and P. Travers, Immuno Biology 3:9 (Garland Publishing Inc., 2nd ed. 1996)). Because folded proteins have complex surfaces, the number of epitopes available is quite numerous; however, due to the conformation of the protein and steric hinderances, the number of antibodies that actually bind to the epitopes is less than the number of available epitopes (C. A. Janeway, Jr. and P. Travers, Immuno Biology 2:14 (Garland Publishing Inc., 2nd ed. 1996)). Epitopes can be identified by any of the methods known in the art.

Antibodies, including scFV fragments, which bind specifically to PD-1, LAG-3, or ICOS and either block its function ("antagonist antibodies") or activate its function (agonist antibodies) are encompassed by the invention. Such antibodies can be generated by conventional means.

The disclosure encompasses monoclonal antibodies against PD-1, LAG-3, or ICOS that block its function ("antagonist antibodies") or activate its function (agonist antibodies). Exemplary blocking monoclonal antibodies against PD-1, LAG-3, and ICOS are described in WO 2012/131004 and WO 2011/041613.

Antibodies are capable of binding to their targets with both high avidity and specificity. They are relatively large molecules (∼150kDa), which can sterically inhibit interactions between two proteins (e.g. PD-1, LAG-3, or ICOS and its target ligand) when the antibody binding site falls within proximity of the protein-protein interaction site. The invention further encompasses antibodies that bind to epitopes within close proximity to a PD-1, LAG-3, or ICOS -ligand binding site.

In various embodiments, the invention encompasses antibodies that interfere with intermolecular interactions (e.g. protein-protein interactions), as well as antibodies that perturb intramolecular interactions (e.g. conformational changes within a molecule). Antibodies can be screened for the ability to block the biological activity of PD-1 or LAG-3, or the binding of PD-1 or LAG-3 to a ligand, and/or for other properties. Agonist antibodies can further be screened for the ability to activate the biological activity of ICOS.

Both polyclonal and monoclonal antibodies can be prepared by conventional techniques.

PD-1, LAG-3, and ICOS and peptides based on the amino acid sequence of PD-1, LAG-3, and ICOS, can be utilized to prepare antibodies that specifically bind to PD-1, LAG-3, or ICOS. The term "antibodies" is meant to include polyclonal antibodies, monoclonal antibodies, fragments thereof, such as F(ab')2 and Fab fragments, single-chain variable fragments (scFvs), single-domain antibody fragments (VHHs or Nanobodies), bivalent antibody fragments (diabodies), as well as any recombinantly and synthetically produced binding partners.

Antibodies are defined to be specifically binding if they bind PD-1, LAG-3, or ICOS polypeptide with a Ka of greater than or equal to about 10⁷ M⁻¹. Affinities of binding partners or antibodies can be readily determined using conventional techniques, for example those described by Scatchard et al., Ann. N.Y. Acad. Sci., 51:660 (1949).

Polyclonal antibodies can be readily generated from a variety of sources, for example, horses, cows, goats, sheep, dogs, chickens, rabbits, mice, or rats, using procedures that are well known in the art. In general, purified PD-1, LAG-3, or ICOS or a peptide based on the amino acid sequence of PD-1, LAG-3, or ICOS that is appropriately conjugated is administered to the host animal typically through parenteral injection. The immunogenicity of PD-1, LAG-3, or ICOS can be enhanced through the use of an adjuvant, for example, Freund's complete or incomplete adjuvant. Following booster immunizations, small samples of serum are collected and tested for reactivity to PD-1, LAG-3, or ICOS polypeptide. Examples of various assays useful for such determination include those described in Antibodies: A Laboratory Manual, Harlow and Lane (eds.), Cold Spring Harbor Laboratory Press, 1988; as well as procedures, such as countercurrent immuno-electrophoresis (CIEP), radioimmunoassay, radioimmunoprecipitation, enzyme-linked immunosorbent assays (ELISA), dot blot assays, and sandwich assays. See U.S. Pat. Nos. 4,376,110 and 4,486,530.

Monoclonal antibodies can be readily prepared using well known procedures. See, for example, the procedures described in U.S. Pat. Nos. RE 32,011, 4,902,614, 4,543,439, and 4,411,993; Monoclonal Antibodies, Hybridomas: A New Dimension in Biological Analyses, Plenum Press, Kennett, McKeam, and Bechtol (eds.), 1980.

For example, the host animals, such as mice, can be injected intraperitoneally at least once and preferably at least twice at about 3 week intervals with isolated and purified PD-1, LAG-3, or ICOS or conjugated PD-1, LAG-3, or ICOS peptide, optionally in the presence of adjuvant. Mouse sera are then assayed by conventional dot blot technique or antibody capture (ABC) to determine which animal is best to fuse. Approximately two to three weeks later, the mice are given an intravenous boost of PD-1, LAG-3, or ICOS or conjugated PD-1, LAG-3, or ICOS peptide. Mice are later sacrificed and spleen cells fused with commercially available myeloma cells, such as Ag8.653 (ATCC), following established protocols. Briefly, the myeloma cells are washed several times in media and fused to mouse spleen cells at a ratio of about three spleen cells to one myeloma cell. The fusing agent can be any suitable agent used in the art, for example, polyethylene glycol (PEG). Fusion is plated out into plates containing media that allows for the selective growth of the fused cells. The fused cells can then be allowed to grow for approximately eight days. Supernatants from resultant hybridomas are collected and added to a plate that is first coated with goat anti-mouse Ig. Following washes, a label, such as a labeled PD-1, LAG-3, or ICOS polypeptide, is added to each well followed by incubation. Positive wells can be subsequently detected. Positive clones can be grown in bulk culture and supernatants are subsequently purified over a Protein A column (Pharmacia).

The monoclonal antibodies of the invention can be produced using alternative techniques, such as those described by Alting-Mees et al., "Monoclonal Antibody Expression Libraries: A Rapid Alternative to Hybridomas", Strategies in Molecular Biology 3:1-9 (1990). Similarly, binding partners can be constructed using recombinant DNA techniques to incorporate the variable regions of a gene that encodes a specific binding antibody. Such a technique is described in Larrick et al., Biotechnology, 7:394 (1989).

Antigen-binding fragments of such antibodies, which can be produced by conventional techniques, are also encompassed by the present invention. Examples of such fragments include, but are not limited to, Fab and F(ab')2 fragments. Antibody fragments and derivatives produced by genetic engineering techniques are also provided.

The monoclonal antibodies of the present invention include chimeric antibodies, e.g., humanized versions of murine monoclonal antibodies. Such humanized antibodies can be prepared by known techniques, and offer the advantage of reduced immunogenicity when the antibodies are administered to humans. In one embodiment, a humanized monoclonal antibody comprises the variable region of a murine antibody (or just the antigen binding site thereof) and a constant region derived from a human antibody. Alternatively, a humanized antibody fragment can comprise the antigen binding site of a murine monoclonal antibody and a variable region fragment (lacking the antigen-binding site) derived from a human antibody. Procedures for the production of chimeric and further engineered monoclonal antibodies include those described in Riechmann et al. (Nature 332:323, 1988), Liu et al. (PNAS 84:3439, 1987), Larrick et al. (Bio/Technology 7:934, 1989), and Winter and Harris (TIPS 14:139, May, 1993). Procedures to generate antibodies transgenically can be found in GB 2,272,440, U.S. Pat. Nos. 5,569,825 and 5,545,806.

Antibodies produced by genetic engineering methods, such as chimeric and humanized monoclonal antibodies, comprising both human and non-human portions, which can be made using standard recombinant DNA techniques, can be used. Such chimeric and humanized monoclonal antibodies can be produced by genetic engineering using standard DNA techniques known in the art, for example using methods described in Robinson et al. International Publication No. WO 87/02671; Akira, et al. European Patent Application 0184187; Taniguchi, M., European Patent Application 0171496; Morrison et al. European Patent Application 0173494; Neuberger et al. PCT International Publication No. WO 86/01533; Cabilly et al. U.S. Pat. No. 4,816,567; Cabilly et al. European Patent Application 0125023; Better et al., Science 240:1041 1043, 1988; Liu et al., PNAS 84:3439 3443, 1987; Liu et al., J. Immunol. 139:3521 3526, 1987; Sun et al. PNAS 84:214 218, 1987; Nishimura et al., Canc. Res. 47:999 1005, 1987; Wood et al., Nature 314:446 449, 1985; and Shaw et al., J. Natl. Cancer Inst. 80:1553 1559, 1988); Morrison, S. L., Science 229:1202 1207, 1985; Oi et al., BioTechniques 4:214, 1986; Winter U.S. Pat. No. 5,225,539; Jones et al., Nature 321:552 525, 1986; Verhoeyan et al., Science 239:1534, 1988; and Beidler et al., J. Immunol. 141:4053 4060, 1988.

In connection with synthetic and semi-synthetic antibodies, such terms are intended to cover but are not limited to antibody fragments, isotype switched antibodies, humanized antibodies (e.g., mouse-human, human-mouse), hybrids, antibodies having plural specificities, and fully synthetic antibody-like molecules.

For therapeutic applications, "human" monoclonal antibodies having human constant and variable regions are often preferred so as to minimize the immune response of a patient against the antibody. Such antibodies can be generated by immunizing transgenic animals which contain human immunoglobulin genes. See Jakobovits et al. Ann NY Acad Sci 764:525-535 (1995).

Human monoclonal antibodies against PD-1, LAG-3, or ICOS polypeptides can also be prepared by constructing a combinatorial immunoglobulin library, such as a Fab phage display library or a scFv phage display library, using immunoglobulin light chain and heavy chain cDNAs prepared from mRNA derived from lymphocytes of a subject. See, e.g., McCafferty et al. PCT publication WO 92/01047; Marks et al. (1991) J. Mol. Biol. 222:581 597; and Griffths et al. (1993) EMBO J 12:725 734. In addition, a combinatorial library of antibody variable regions can be generated by mutating a known human antibody. For example, a variable region of a human antibody known to bind PD-1, LAG-3, or ICOS, can be mutated, by for example using randomly altered mutagenized oligonucleotides, to generate a library of mutated variable regions which can then be screened to bind to PD-1, LAG-3, or ICOS. Methods of inducing random mutagenesis within the CDR regions of immunoglobin heavy and/or light chains, methods of crossing randomized heavy and light chains to form pairings and screening methods can be found in, for example, Barbas et al. PCT publication WO 96/07754; Barbas et al. (1992) Proc. Nat'l Acad. Sci. USA 89:4457 4461.

An immunoglobulin library can be expressed by a population of display packages, preferably derived from filamentous phage, to form an antibody display library. Examples of methods and reagents particularly amenable for use in generating antibody display library can be found in, for example, Ladner et al. U.S. Pat. No. 5,223,409; Kang et al. PCT publication WO 92/18619; Dower et al. PCT publication WO 91/17271; Winter et al. PCT publication WO 92/20791; Markland et al. PCT publication WO 92/15679; Breitling et al. PCT publication WO 93/01288; McCafferty et al. PCT publication WO 92/01047; Garrard et al. PCT publication WO 92/09690; Ladner et al. PCT publication WO 90/02809; Fuchs et al. (1991) Bio/Technology 9:1370 1372; Hay et al. (1992) Hum Antibod Hybridomas 3:81 85; Huse et al. (1989) Science 246:1275 1281; Griffths et al. (1993) supra; Hawkins et al. (1992) J Mol Biol 226:889 896; Clackson et al. (1991) Nature 352:624 628; Gram et al. (1992) PNAS 89:3576 3580; Garrad et al. (1991) Bio/Technology 9:1373 1377; Hoogenboom et al. (1991) Nuc Acid Res 19:4133 4137; and Barbas et al. (1991) PNAS 88:7978 7982. Once displayed on the surface of a display package (e.g., filamentous phage), the antibody library is screened to identify and isolate packages that express an antibody that binds a PD-1, LAG-3, or ICOS polypeptide. In a preferred embodiment, the primary screening of the library involves panning with an immobilized PD-1, LAG-3, or ICOS polypeptide and display packages expressing antibodies that bind immobilized PD-1, LAG-3, or ICOS polypeptide are selected.

### Exemplary Combination therapies with recombinant orthopoxvirus-expressing a tumor antigen and agonists/antagonists of PD-1, LAG-3, and ICOS

In at least one aspect, the present invention encompasses medicament or composition for use in the treatment of a human cancer patient employing combinations of a recombinant orthopoxvirus and/or avipoxvirus each comprising a nucleic acid encoding a tumor antigen with one or more antibodies, agonists, or antagonists according to the invention.

In one exemplary embodiment, patients with a cancer mediated by cells overexpressing the tumor-associated antigen HER-2 (e.g., breast cancer) can be treated by the combination of: 1) one or more recombinant orthopoxviruses, for example a vaccinia virus (e.g., Wyeth or MVA) encoding a HER-2 antigen or 2) the combination of a recombinant orthopoxvirus and a recombinant an avipoxvirus (e.g., fowlpoxvirus, POXVAC-TC), encoding a HER-2 antigen; with combination 1) or 2) being administered with one or more antibodies, agonists, and/or antagonists according to the invention. In a preferred embodiment, the MVA is MVA-BN. In a particularly preferred embodiment, the MVA encodes a polypeptide comprising SEQ ID NO:2.

In an additional exemplary embodiments, patients with prostate cancer can be treated by the combination of a recombinant orthopoxvirus, for example a vaccinia virus (e.g., Wyeth or MVA) and a recombinant avipoxvirus (e.g., fowlpoxvirus, POXVAC-TC), encoding a PSA and/or PAP antigen, with one or more antibodies, agonists, or antagonists according to the invention. In a particularly preferred embodiment, the combination of the recombinant vaccinia virus and fowlpox virus is PROSTVAC® (vaccinia virus and fowlpox virus, each expressing PSA and TRICOM) and is administered with one or more antibodies, agonists, and/or antagonists according to the invention.

In still an additional embodiment, patients with cancer mediated by cells overexpressing the tumor-associated antigen CEA and/or MUC-1 can be treated by the combination of a recombinant orthopoxvirus, for example a vaccinia virus (e.g., Wyeth, MVA, or MVA-BN) and a recombinant avipoxvirus (e.g., fowlpoxvirus, POXVAC-TC), each encoding CEA and MUC-1 antigen, with one or more antibodies, agonists, or antagonists according to the invention. Some non-limiting examples of cancers mediated by cells overexpressing CEA and MUC-1 include, but are not limited to, breast cancer, colorectal cancer, lung cancer, gastric cancer, pancreatic cancer, bladder cancer, and ovarian cancer. In one or more preferred embodiments, the combination therapy includes CV301 (vaccinia virus and fowlpox virus, each expressing CEA, MUC-1, and TRICOM). In another preferred embodiment, the combination therapy includes MVA/Fowlpox-CV301, a heterologous prime-boost that includes MVA (or MVA-BN) and Fowlpox each expressing CEA, MUC-1, and TRICOM.

In yet another embodiment, patients with cancer mediated by cells overexpressing the tumor-associated antigen CEA and/or MUC-1 can be treated by a homologous prime-boost encoding CEA and/or MUC-1 antigens in combination with one or more antibodies, agonists, or antagonists according to the invention. It is contemplated that the two or more recombinant orthopoxviruses can be a vaccinia virus (e.g., Wyeth, MVA, or MVA-BN). Some non-limiting examples of cancers mediated by cells overexpressing CEA and MUC-1 include, but are not limited to, breast cancer, colorectal cancer, lung cancer, gastric cancer, pancreatic cancer, bladder cancer, and ovarian cancer. In one or more preferred embodiments, the treatment can be MVA-CV301, which includes two or more MVA (or MVA-BN) viruses each expressing CEA, MUC-1, and TRICOM) administered as a homologous prime-boost.

The recombinant orthopoxvirus and/or avipoxvirus can be administered either systemically or locally, i.e., by parenteral, subcutaneous, intravenous, intramuscular, intranasal, intradermal, scarification, or any other path of administration known to a skilled practitioner. Preferably, the administration is via scarification. More preferably, the administration is via subcutaneous or intramuscular. In one embodiment, 10⁵-10¹⁰ TCID₅₀ of the recombinant orthopoxvirus and/or avipoxvirus are administered to the patient. Preferably, 10⁷-10¹⁰ TCID₅₀ of the recombinant orthopoxvirus and/or avipoxvirus are administered to the patient. More preferably, 10⁸-10¹⁰ TCID₅₀ of the recombinant orthopoxvirus and/or avipoxvirus are administered to the patient. Most preferably, 10⁸-10⁹ TCID₅₀ of the recombinant orthopoxvirus and/or avipoxvirus are administered to the patient.

It is possible to induce an immune response with a single administration of the recombinant orthopoxvirus and/or avipoxvirus as defined above. The orthopoxvirus and/or avipoxvirus according to the present invention may also be used for a first vaccination and to boost the immune response generated in the first vaccination by administration of the same or a related recombinant orthopoxvirus and/or avipoxvirus than the one used in the first vaccination. The recombinant orthopoxvirus and/or avipoxvirus according to the present invention may also be used in heterologous prime-boost regimes in which one or more of the priming vaccinations is done with an orthopoxvirus as defined above and in which one or more of the boosting vaccinations is done with another type of vaccine, e.g. an avipoxvirus as defined herein or another virus vaccine, a protein or a nucleic acid vaccine.

The cancer preferably includes, but is not limited to, breast cancer, colorectal cancer, lung cancer, gastric cancer, pancreatic cancer, bladder cancer, prostate cancer, and ovarian cancer.

The cancer patient can be any mammal, including a mouse or rat. Preferably, the cancer patient is a primate, most preferably, a human.

In one embodiment, one or more antibodies, agonists or antagonists, according to the invention and the orthopoxvirus encoding a polypeptide comprising a tumor antigen are administered at the same time. The combination treatment is superior to either treatment alone.

In preferred embodiments, the orthopoxvirus is for administration within 1, 2, 3, 4, 5, 6, or 7, days of agonist and/or antagonist administration. The orthopoxvirus can be administered before or after the agonist and/or antagonist.

The dosage agonist or antagonist administered to a patient is typically 0.1 mg/kg to 100 mg/kg of the patient's body weight. Preferably, the dosage administered to a patient is between 0.1 mg/kg and 20 mg/kg of the patient's body weight, more preferably 1 mg/kg to 10 mg/kg of the patient's body weight, most preferably 3 mg/kg to 10 mg/kg of the patient's body weight. Generally, human and humanized antibodies have a longer half-life within the human body than antibodies from other species due to the immune response to the foreign polypeptides. Thus, lower dosages of human antibodies and less frequent administration is often possible.

The quantities of active ingredient necessary for effective therapy will depend on many different factors, including means of administration, target site, physiological state of the patient, and other medicaments administered. Thus, treatment dosages should be titrated to optimize safety and efficacy. Typically, dosages used in vitro can provide useful guidance in the amounts useful for in situ administration of the active ingredients. Animal testing of effective doses for treatment of particular disorders will provide further predictive indication of human dosage. Various considerations are described, for example, in Goodman and Gilman's the Pharmacological Basis of Therapeutics, 7th Edition (1985), MacMillan Publishing Company, New York, and Remington's Pharmaceutical Sciences 18th Edition, (1990) Mack Publishing Co, Easton Pa. Methods for administration are discussed therein, including oral, intravenous, intraperitoneal, intramuscular, transdermal, nasal, iontophoretic administration, and the like.

The compositions of the invention can be administered in a variety of unit dosage forms depending on the method of administration. For example, unit dosage forms suitable for oral administration include solid dosage forms such as powder, tablets, pills, capsules, and dragees, and liquid dosage forms, such as elixirs, syrups, and suspensions. The active ingredients can also be administered parenterally in sterile liquid dosage forms. Gelatin capsules contain the active ingredient and as inactive ingredients powdered carriers, such as glucose, lactose, sucrose, mannitol, starch, cellulose or cellulose derivatives, magnesium stearate, stearic acid, sodium saccharin, talcum, magnesium carbonate and the like. Examples of additional inactive ingredients that can be added to provide desirable color, taste, stability, buffering capacity, dispersion or other known desirable features are red iron oxide, silica gel, sodium lauryl sulfate, titanium dioxide, edible white ink and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric-coated for selective disintegration in the gastrointestinal tract. Liquid dosage forms for oral administration can contain coloring and flavoring to increase patient acceptance.

The concentration of the compositions of the invention in the pharmaceutical formulations can vary widely, i.e., from less than about 0.1%, usually at or at least about 2% to as much as 20% to 50% or more by weight, and will be selected primarily by fluid volumes, viscosities, etc., in accordance with the particular mode of administration selected.

For solid compositions, conventional nontoxic solid carriers can be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more compositions of the invention of the invention, and more preferably at a concentration of 25%-75%.

For aerosol administration, the compositions of the invention are preferably supplied in finely divided form along with a surfactant and propellant. Preferred percentages of compositions of the invention are 0.01%-20% by weight, preferably 1-10%. The surfactant must, of course, be nontoxic, and preferably soluble in the propellant. Representative of such agents are the esters or partial esters of fatty acids containing from 6 to 22 carbon atoms, such as c-aproic, octanoic, lauric, palmitic, stearic, linoleic, linolenic, olesteric and oleic acids with an aliphatic polyhydric alcohol or its cyclic anhydride. Mixed esters, such as mixed or natural glycerides can be employed. The surfactant can constitute 0.1%-20% by weight of the composition, preferably 0.25-5%. The balance of the composition is ordinarily propellant. A carrier can also be included, as desired, as with, e.g., lecithin for intranasal delivery.

The constructs of the invention can additionally be delivered in a depot-type system, an encapsulated form, or an implant by techniques well-known in the art. Similarly, the constructs can be delivered via a pump to a tissue of interest.

Any of the foregoing formulations can be appropriate in treatments and therapies in accordance with the present invention, provided that the active agent in the formulation is not inactivated by the formulation and the formulation is physiologically compatible.

### Therapeutic Compositions and Uses

The present invention further relates to the use of the orthopoxvirus vectors and avipoxvirus vectors according to the invention in the treatment of a human cancer patient which are capable of inducing or contributing to the occurrence or improvement of an immunological reaction against tumor epitopes. The present invention thus provides viruses or vectors that are useful as a medicament or vaccine.

Accordingly, the invention relates to an immunogenic composition comprising a recombinant orthopoxvirus vector (such as vaccinia, MVA, or MVA-BN) and/or a recombinant avipoxvirus vector according to the invention in combination with one or more antibodies, agonists, or antagonists according to the invention.

Thus, the recombinant orthopoxvirus vector and recombinant avipoxvirus vectors according to the invention can be used for the treatment of cancer.

In one or more preferred embodiments, the invention encompasses use of a composition for administration to or treatment of a cancer patient, particularly a breast cancer patient or a prostate cancer patient.

In one embodiment the composition for simultaneous or sequential administration comprising an MVA vector according to the invention and one or more antibody, agonist, or antagonist according to the invention.

The compositions and methods described herein additionally or alternatively can comprise one or more immunostimulatory/regulatory molecules. Any suitable immunostimulatory/regulatory molecule can be used, such as interleukin (IL)-2, IL-4, IL-6, IL-12, IL-15, IL-15/IL-15Ra, IL-15/IL-15Ra-Fc, interferon (IFN)-γ, tumor necrosis factor (TNF)-α, B7.1, B7.2, ICAM-1, ICAM-2, LFA-1, LFA-2, LFA-3, CD70, CD-72, RANTES, G-CSF, GM-CSF, OX-40L, 41 BBL, anti-CTLA-4, IDO inhibitor, anti-PDLl, anti-PDl, and combinations thereof. Preferably, the composition comprises a combination of B7.1, ICAM-1, and LFA-3 (also referred to as TRICOM). The one or more immunostimulatory/regulatory molecules can be administered in the form of a vector (e.g., a recombinant viral vector, such as a poxvirus vector) comprising a nucleic acid encoding one or more immunostimulatory/regulatory molecules. For example, the one or more immunostimulatory/regulatory molecules (e.g., IL-12) can be administered in the form of a DNA plasmid with or without chitosan.

In a more preferred embodiment, in addition to comprising at least one tumor antigen, the recombinant orthopoxvirus and/or the recombinant avipoxvirus comprise one or more nucleic acids encoding for the combination of B7.1, ICAM-1, and/or LFA-3 (also referred to as TRICOM).

### EXAMPLES

### Example 1

### Construction of MVA-BN-mHER2

Simultaneous infection and transfection of cultures allowed homologous recombination to occur between the viral genome and the recombination plasmid. Insert-carrying virus was isolated, characterized, and virus stocks were prepared.

Plasmid pBN279 contains sequences which are also present in MVA-BN (the Intergenic Region between ORF 64 and 65, IGR 64/65). The mHER2 sequence was inserted between the MVA-BN sequences to allow for recombination into the MVA-BN viral genome. Thus, a plasmid was constructed that contained the mHER2 sequence downstream of a poxvirus promoter, specifically the synthetic vaccinia virus promoter (PrS). The plasmid also contained a selection cassette comprising the PrS promoter upstream of a drug resistance gene (guanine-xanthine phosphoribosyltransferase; EcoGPT) and a PrS promoter upstream of monomeric Red Fluorescence Protein 1 (mRFP1).

The HER-2 sequence was modified by addition of nucleotides sequences encoding tetanus toxin epitopes of p2 and p30 to increase the immune response against it (mHER2). After mHER2 was inserted into the MVA-BN genome, and the selection cassette was removed, the virus "insert region" had the following structure (shown in the opposite orientation compared to the surrounding viral reading frame):
PrS promoter - mHER2 sequence. The insert region was flanked by MVA-BN intergenic region sequences 64/65 (Flank 1(64) and Flank 2 (65)) as in the bacterial recombination plasmid pBN279. The nucleotide sequence of the construct is shown below.

### (SEQ ID NO:1).

HER2 start and stop codons are indicated in bold. Flanking sequences are indicated in italics. Flank 1 (64): 451-1052, underlined HER2: 3298-1247 (bold), start: 3298-3296 (bold + underlined), stop: 1249-1247 (bold + underlined), PrS promoter: 3442-3403 (italic + underlined), Flank (65): 3463-4065 (underlined).

Translation of the encoded mHER2 polypeptide is shown below:

### (SEQ ID NO:2).

The tetanus toxin epitopes of p2 and p30 sequences are indicated in bold.

CEF cultures were inoculated with MVA-BN and also transfected with pBN279 plasmid DNA. In turn, samples from these cell cultures were inoculated into CEF cultures in medium containing selection drugs, and mRFP1-expressing viral clones were isolated by plaque purification. Virus stocks which grew in the presence of the selection drugs and expressed mRFP1 were designated MVA-BN-mHER2. Generation of MVA-BN-mHER2 and preparation of the virus stock involved nine (9) sequential passages, including four (4) plaque purifications.

MVA-BN-mHER2 was passaged in CEF cell cultures in the absence of selection drugs. The absence of selection drugs allowed loss of the region encoding the selection genes, gpt and mRFP1 and the associated promoters (the selection cassette) from the inserted sequence. Recombination resulting in loss of the selection cassette is mediated by the Flank 1 (F1) region and a subsection of that region, the F1 repeat (F1 rpt), which flank the selection cassette in plasmid pBN279. These duplicated sequences were included to mediate recombination that results in loss of the selection cassette, leaving only the mHER2 sequence inserted in the 64/65 intergenic region.

Plaque-purified virus lacking the selection cassette was prepared. Such preparation involved fifteen (15) passages including five (5) plaque purifications.

The presence of the mHER2 sequence and absence of parental MVA-BN virus in MVA-BN-mHER2 stocks was confirmed by PCR analysis, and nested PCR was used to verify the absence of the selection cassette (the gpt and mRFP1 genes).

Expression of the mHER2 protein was demonstrated in cells inoculated with MVA-BN-mHER2 *in vitro.*

### Example 2

### Induction of an anti-tumor response in mice treated with MVA-BN-HER2 and antibodies

Female BALB/c mice (6-8 weeks old, ∼ 20 g) were purchased from Simonsen Laboratories, Gilroy, CA. For the experimental lung metastasis model, mice were implanted i.v. with 5.0×10⁴ CT26-HER-2 cells in 300 µL DPBS which forms tumors in the lungs. In the solid tumor model, mice were implanted i.d. in the back with 1.0×10⁵ CT26-HER-2 cells in 100 µL DPBS. Tumors were measured twice weekly and the tumor volume calculated according to the formula: tumor volume (mm³) = (length × width²)/2.

The following antibodies were purchased from Bio X Cell (West, Lebanon, NH): anti-ICOS (Clone 17G9), anti-CTLA-4 (9D9), anti-PD-1 (RMP1-14), and anti-LAG-3 (C9B7W). All antibodies were injected i.p. at 200 µg per mouse in 100 µL PBS on the days indicated in the figure legends. For virus treatments, mice were treated with 7.1 µL of 1.0×10⁷ Inf. U. MVA-BN-mHER2 by tail scarification (t.s., produced by Bavarian Nordic [BN], Martinsried, Germany).

Serum antibody titers were determined by ELISA, and IFN-γ by ELISPOT as described in Mandl et al., Cancer Immunol Immunother (2012) 61:19-29. Whole blood, spleens, and lungs were collected for FACS analysis. Lungs were cut to 1-2 mm pieces and incubated for 1 h at 37 °C in DMEM with 10% FBS, 50 U/mL DNAse I and 250 U/mL Collagenase I (Worthington Biochemical Corporation, Lakewood, NJ). Red blood cells from the lungs, splenocytes, and whole blood were lysed and single cell suspensions were stained according to standard protocols with antibodies purchased from Biolegend (San Diego, CA): CD3e (145-2c11 or 500A2), CD4 (RM4-5), CD8 (53-6.7), CD278 (ICOS, 7E.17G9), CD279 (PD-1, 29F.1AA12), and CD223 (LAG-3, C9B7W). Regulatory T-cells were identified using the FoxP3/Transcription Factor Staining Buffer Set and FoxP3 antibody (FJK-16s) according to the manufacturer's instructions (eBioscience, San Diego, CA).

All statistical analysis was performed as described in the figure legends using GraphPad Prism version 6.01 for Windows (GraphPad Software, La Jolla, CA).

### Example 3

### MVA-BN-HER2 Treatment Increases ICOS on CD8⁺ and CD4⁺ T Cells

Naive, tumor free mice were treated with MVA-BN-mHER2 (1E7 Inf.U. t.s.) on day 1 or days 1 and 15. Organs from 3 mice at each time point. Shown in Figure 1, treatment with MV-BN-mHER2 increased ICOS expression on CD8+ and CD4+ T Cells.

### Example 4

### Induction of an anti-tumor response in mice treated with MVA-BN-HER2 with anti-ICOS.

Balb/c mice were implanted on day 1 with 1E5 CT26-HER2 cells i.d. Mice were treated with 1E7 Inf.U. MVA-BN-mHER2 by tail scarification (t.s.) on days 1 and 15, and anti-ICOS on days 1, 4, 8, 11, 15, 18, 22, and 25 (200, µg i.p.). **, p<0.01, **** p<0.0001, Repeated measures Two-way ANOVA with Tukey's Multiple Comparisons post-test. Shown in Figure 2, tumor volume was significantly decreased with MVA-BN-mHER2 plus anti-ICOS as compared to MVA-BN-mHER2 alone.

### Example 5

### Induction of an anti-tumor response in mice treated with MVA-BN-HER2 with anti-CTLA-4

In a CT26-HER-2 experimental lung metastasis model, mice were implanted on day 1 with 5E4 CT26-HER-2 cells i.v. which forms tumors in the lungs. Mice were treated with MVA-BN-mHER2 (1E7 Inf.U. t.s.) on days 4 and 18 and anti-CTLA-4 (200 µg, i.p.) on days 3 and 17. **** p<0.0001, Log-Rank Test. Shown in Figure 3, treatment with MVA-BN-mHER2 and anti-CTLA-4 was effective at increasing the overall survival rate.

### Example 6

### MVA-BN-HER2 Significantly Reduces Pulmonary Tumor Burden by Day 25

Mice were implanted i.v. on day 1 with 5E4 CT26-HER-2 cells i.v. which forms tumors in the lungs. Mice were treated with MVA-BN-mHER2 (1E7 Inf.U. t.s.) on day 4 and 18 and anti-CTLA-4 (200 µg, i.p.) on days 3 and 17. A) On day 25, mice were euthanized and perfused through the trachea with Trypan Blue. Lungs were removed and briefly submerged in Hydrogen Peroxide and washed in PBS. Tumors visible as small masses in Untreated and anti-CTLA-4 treated mice. There were no visible tumors in the lungs of mice treated with MVA-BN-mHER2. Scale bar equals 1 cm. B) Lung weight on day 25. **** p<0.0001, One-Way ANOVA with Dunnett's Multiple Comparisons test. The results are shown in Figure 4.

### Example 7

### Pulmonary ICOS increases with MVA-BN-HER2 Treatment or Pulmonary Tumors

Mice were implanted i.v. on day 1 with 5E4 CT26-HER-2 cells i.v. which forms tumors in the lungs. Mice were treated with MVA-BN-mHER2 (1E7 Inf.U. t.s.) on day 4 and 18 and anti-CTLA-4 (200 µg, i.p.) on days 3 and 17. Organs from 3 mice at each time point were pooled for analysis (A and B). Data shown as mean ± SEM from three independent experiments with 3-4 mice per group (C and D). Shown in Figure 5, Pulmonary ICOS increased upon treatment with MVA-BN-mHER2.

### Example 8

### In Tumor Bearing Mice, ICOS⁺ CD4⁺ T Cells are FoxP3⁺

Mice were implanted i.v. on day 1 with 5E4 CT26-HER-2 cells i.v. which forms tumors in the lungs. Mice were treated with MVA-BN-mHER2 (1E7 Inf.U. t.s.) on day 4 and 18 and anti-CTLA-4 (200 µg, i.p.) on days 3 and 17. Organs from 3 mice at each time point were pooled for analysis (A and B). Data shown as mean ± SEM from three independent experiments with 3-4 mice per group (C and D). Shown in Figure 6, ICOS expression increased in both FoxP3+ Tregs and FoxP3- Teff cells in tumor bearing control and anti-CTLA-4 treated mice where the tumor burden was high. ICOS increased only on FoxP3- Teff cells following MVA-BN-mHER2 treatment and was more pronounced following combination with anti-CTLA-4.

### Example 9

### MVA-BN-HER2 with anti-CTLA-4 Increases the Effector to Regulatory T Cell Ratio

Mice were implanted i.v. on day 1 with 5E4 CT26-HER-2 cells i.v. which forms tumors in the lungs. Mice were treated with MVA-BN-mHER2 (1E7 Inf.U. t.s.) on days 4 and 18 and anti-CTLA-4 (200 µg, i.p.) on days 3 and 17. Organs from 3 mice at each time point were pooled for analysis (A and B). Data shown as mean ± SEM from three independent experiments with 3-4 mice per group (C and D). Shown in Figure 7, treatment with MVA-BN-mHER2 and anti-CTLA-4 increased both the CD8 and CD4 Effector to Regulatory T cell ratio in the tumor site, as well as the spleen and blood.

### Example 10

### PD-1 Expression Increases with MVA-BN-HER2 Treatment

Naive, tumor free mice were treated with MVA-BN-HER2 (1E7 Inf.U., t.s.) on day 1 or days 1 and 15. Organs from 3 mice at each time point. Shown in Figure 8, treatment with MV-BN-mHER2 increased PD-1 expression on CD8⁺ and CD4⁺ T Cells.

### Example 11

### Induction of an anti-tumor response in mice treated with MVA-BN-HER2 and anti-PDl

Balb/c mice were implanted on day 1 with 1E5 CT26-HER-2 cells i.d. Mice were treated with 1E7 Inf.U. MVA-BN-mHER2 by tail scarification (t.s.) on days 1 and 15, and anti-PD1 on days 1 and 15 (200 µg i.p.). **** p<0.0001, Repeated measures Two-way ANOVA with Tukey's Multiple Comparisons post-test. Shown in Figure 9, tumor volume was significantly decreased with MVA-BN-mHER2 plus anti-PD1 as compared treatment with antiPD-1 alone, and survival was significantly increased compared to MVA-BN-mHER2 alone.

### Example 12

### LAG-3 Immune Response to MVA-BN-HER2

Naive, tumor free mice were treated with MVA-BN-mHER2 (1E7 Inf.U. t.s.) on day 1 or days 1 and 15. Organs from 3 mice at each time point. Shown in Figure 10, LAG-3 expression on CD8⁺ and CD4⁺ T Cells.

### Example 13

### Induction of an anti-tumor response in mice treated with MVA-BN-HER2 and anti-LAG-3

Balb/c mice were implanted on day 1 with 1E5 CT26-HER-2 cells i.d. Mice were treated with 1E7 Inf.U. MVA-BN-mHER2 by tail scarification (t.s.) on days 1 and 15, and anti-LAG-3 on days 1 and 15 (200 µg i.p.). **** p<0.0001, Repeated measures Two-way ANOVA with Tukey's Multiple Comparisons post-test. Shown in Figure 11, treatment with MVA-BN-mHER2 in combination with anti-LAG3 increased the overall survival rate as compared to MVA-BN-mHER2 alone and anti-LAG3 alone.

### Example 14

### Induction of an anti-tumor response in mice treated with MVA-BN-HER2 and anti-PD-1 and anti-LAG-3

Balb/c mice were implanted on day 1 with 1E5 CT26-HER-2 cells i.d. In Figure 12, mice were treated with 1E7 Inf.U. MVA-BN-mHER2 by tail scarification (t.s.) on days 1 and 15, and anti-PD1 and anti-LAG-3 on days 1 and 15 (200 µg each, i.p.). In Figure 13, mice were treated with 1E7 Inf.U. MVA-BN-mHER2 by t.s. on days 4 and 18, and anti-PD-1 and anti-LAG-3 on days 4 and 18 (200 µg i.p.). **** p<0.0001, Repeated measures Two-way ANOVA with Tukey's Multiple Comparisons post-test. Shown in Figure 12 and 13, treatment with MVA-BN-mHER2 in combination with both anti-PD1 and anti-LAG3, decreased the tumor volume (Figure 12A and 13A) and increased the overall survival rate (Figure 12B and 13B) as compared to MVA-BN-mHER2 alone and anti-PDl and anti-LAG3 alone.

### Example 15

### Induction of an anti-tumor response in mice treated with MVA-BN-HER2 and anti-PD-1 and anti-LAG-3

In a CT26-HER-2 experimental lung metastasis model, mice were implanted on day 1 with 5E4 CT26-HER-2 cells i.v. which form tumors in the lungs. Mice were treated with MVA-BN-mHER2 (1E7 Inf.U. t.s.) on day 4 and 18 and anti-PD-1 and anti-LAG-3 (200 µg each, i.p.) on days 4 and 18. * P<0.05, *** p<0.001, Log-Rank Test. Shown in Figure 14, treatment with MVA-BN-mHER2 in combination with anti-PD-1 and anti-LAG3 increased the overall survival rate as compared to or anti-PDl and anti-LAG3 alone.

### Example 16

### ELISPOT MVA response

Balb/c mice were implanted on day 1 with 1E5 CT26-HER-2 cells i.d. Mice were treated with 1E7 Inf.U. MVA-BN-mHER2 by tail scarification (t.s.) on days 4 and 18, and anti-PD1 and anti-LAG-3 on days 4 and 18 (200 µg i.p.). Four weeks after the last treatment, specific T-cell responses were determined by IFN-γ ELISPOT as described in Mandl et al., Cancer Immunol Immunother (2012) 61:19-29. Shown in Figure 15, treatment with MVA-BN-mHER2 in combination with anti-PD-1 and anti-LAG3 increased tumor antigen specific IFN-γ levels as compared to anti-PD 1 and anti-LAG3 alone.

### Example 17

### Antibody Titers

Balb/c mice were implanted on day 1 with 1E5 CT26-HER-2 cells i.d. Mice were treated with 1E7 Inf.U. MVA-BN-mHER2 by tail scarification (t.s.) on days 4 and 18, and anti-PD1 and anti-LAG-3 on days 4 and 18 (200 µg i.p.). Serum antibody titers were determined by ELISA as described in Mandl et al., Cancer Immunol Immunother (2012) 61:19-29. 2. Results are shown in Figure 16.

### Example 18

### Induction of an anti-tumor response in mice treated with MVA-BN-CV301 and anti-PD-1

Female C57/BL6 mice (6-8 weeks old, -20 g, Simonsen Laboratories, Gilroy CA) were implanted on day 1 with MC38-CEA cells (2×10⁵, i.d. in the back flank). Mice were treated on days 1 and 15 with MVA-BN-CV301 (1E7 Inf.U., s.c. above the tail base). Mice were treated with anti-PD-1 on days 1 and 15 (200 µg i.p.). The results are shown in Figure 17. * p<0.05, **** p<0.0001, Repeated measures Two-way ANOVA with Tukey's Multiple Comparisons post-test.

### Example 19

### Induction of an anti-tumor response in mice treated with MVA-BN-CV301 and anti-LAG-3

Female C57BL/6 mice (6-8 weeks old, -20 g, Simonsen Laboratories, Gilroy CA) were implanted on day 1 with MC38-CEA cells (2×10⁵, i.d. in the back flank). Mice were treated on days 1 and 15 with MVA-BN-CV301 (1E7 Inf.U., s.c. above the tail base). Mice were treated with anti-LAG-3 on days 1 and 15 (200 µg i.p.). The results are shown in Figure 18. ** p<0.01, *** p<0.001, Repeated measures Two-way ANOVA with Tukey's Multiple Comparisons post-test.

### Example 20

### Induction of an anti-tumor response in mice treated with MVA-BN-CV301 and anti-PD-1 and anti-LAG-3

Female C57BL/6 mice (6-8 weeks old, -20 g, Simonsen Laboratories, Gilroy CA) were implanted on day 1 with MC38-CEA cells (2×10⁵, i.d. in the back flank). Mice were treated on days 1 and 15 with MVA-BN-CV301 (1E7 Inf.U., s.c. above the tail base). Mice were treated with anti-PDl and anti-LAG-3 on days 1 and 15 (200 µg i.p. each). ** p<0.01, **** p<0.0001, Repeated measures Two-way ANOVA with Tukey's Multiple Comparisons post-test. Shown in Figure 19, the combination treatment of MVA-BN CV301 and anti-PD1 and anti-LAG3 resulted in a decrease in tumor volume as compared to anti-PDl and anti-LAG3 alone or MVA-BN-CV301 alone.

### Example 21

### Induction of an anti-tumor response in mice treated with PROSTVAC and anti-PD-1

Male BALB/c mice (6-8 weeks old, -20 g, Simonsen Laboratories, Gilroy CA) were implanted on day 1 with E6 cells (RM11-PSA, 1.5×10⁵, i.d. in the back flank). Mice were treated on day 1 with PROSTVAC-V (2E7 Inf. U., s.c. at the tail base), and on days 8 and 15 with PROSTVAC-F (1E8 Inf. U., s.c. at the tail base). Mice were treated on days 1 and 15 with anti-PD-1 (200 µg i.p.). The results of the treatment are shown in Figure 20.

### Example 22

### Induction of an anti-tumor response in mice treated with PROSTVAC and anti-LAG-3

Male BALB/c mice (6-8 weeks old, -20 g, Simonsen Laboratories, Gilroy CA) were implanted on day 1 with E6 cells (RM11-PSA, 1.5×10⁵, i.d. in the back flank). Mice were treated on day 1 with PROSTVAC-V (2E7 Inf. U., s.c. at the tail base), and on days 8 and 15 with PROSTVAC-F (1E8 Inf. U., s.c. at the tail base). Mice were treated on days 1 and 15 with anti-LAG-3 (200 µg i.p.). The results of the treatment are shown in Figure 21.

### Example 23

### Induction of an anti-tumor response in mice treated with PROSTVAC and anti-PD-1 and anti-LAG-3

Male BALB/c mice (6-8 weeks old, -20 g, Simonsen Laboratories, Gilroy CA) were implanted on day 1 with E6 cells (RM11-PSA, 1.5×10⁵, i.d. in the back flank). Mice were treated on day 1 with PROSTVAC-V (2E7 Inf. U., s.c. at the tail base), and on days 8 and 15 with PROSTVAC-F (1E8 Inf. U., s.c. at the tail base). Mice were treated with on days 1 and 15 with anti-PD-1 and anti-LAG-3 (200 µg each, i.p.). Shown in Figure 22, the combination treatment of PROSTVAC with anti-PD-1 and anti-LAG-3 resulted in a decrease in tumor volume as compared to anti-PD-1 and anti-LAG-3 alone or PROSTVAC alone.

### Example 24

### Induction of an anti-tumor response in mice treated with PANVAC (CV301-V/F) and anti-PD-1

Female C57/BL6 mice transgenic for human CEA ((Tg(CEA)18/B6j, received from Jack Shively from the City of Hope National Medical Center, see also Clarke et a. Cancer Research, 58:1469ff., 1998) were implanted on day 1 with MC38-CEA cells (3.0×10⁵, i.d. in the back flank). Mice were treated on day 4 with CV301-Vaccinia (CV301-V) (also known as PANVAC-V) (2E7 Inf. U., s.c. at the tail base), and on days 11 and 18 with CV301-Fowlpox (CV-301-F) (also known as PANVAC-F)(1E8 Inf. U., s.c. at the tail base). CV301-V/F treatments were admixed with Fowlpox GM-CSF (1E7 Inf. U.) on days 4, 11, and 18. Mice were treated with on days 4, 11, and 18 with anti-PD-1 (200 µg i.p.). ** p<0.01, **** p<0.0001, Repeated measures Two-way ANOVA with Tukey's Multiple Comparisons post-test. Shown in Figure 23, the combination treatment of CV301-V/F with anti-PD-1 delayed tumor growth compared to control mice.

## Claims

1. A medicament or composition for use in the treatment of a human cancer patient, comprising: (a) a first recombinant orthopoxvirus comprising a nucleic acid encoding a polypeptide of a CEA antigen and a nucleic acid encoding a polypeptide of a MUC-1 antigen; and (b) at least one of an antagonist of PD-1 that is an antibody and an antagonist of LAG-3 that is an antibody ; wherein (a) and (b) are to be administered as a combination.

2. The medicament or composition for use of claim 1, wherein the recombinant orthopoxvirus is selected from a vaccinia virus, a modified vaccinia Ankara (MVA), and MVA-BN.

3. The medicament or composition for use of claims 1-2, further comprising: (c) a recombinant avipoxvirus comprising a nucleic acid encoding a polypeptide of at least one tumor antigen; and (d) at least one of an antagonist of PD-1 that is an antibody and an antagonist of LAG-3 that is an antibody; wherein (c) and (d) are to be administered as a combination.

4. The medicament or composition for use of claim 3, wherein the recombinant avipoxvirus and antagonist combination is to be administered after the recombinant orthopoxvirus and antagonist combination of claim 1.

5. The medicament or composition for use of claim 3-4, wherein the avipoxvirus is a fowlpoxvirus.

6. The medicament or composition for use of claim 1-2, further comprising: (e) two or more recombinant orthopoxviruses comprising a nucleic acid encoding a polypeptide of at least one tumor antigen; and (f) at least one of an antagonist of PD-1 that is an antibody and an antagonist of LAG-3 that is an antibody; wherein (e) and (f)are to be administered as a combination treatment.

7. The medicament or composition for use of claims 1-6, wherein the cancer is breast cancer, lung cancer, colorectal cancer, gastric cancer, pancreatic cancer, prostate cancer, bladder cancer, or ovarian cancer.

## Patentansprüche

1. Arzneimittel oder Zusammensetzung zur Verwendung bei der Behandlung eines menschlichen Krebspatienten, umfassend: (a) ein erstes rekombinantes Orthopoxvirus, das eine Nukleinsäure, die ein Polypeptid eines CEA-Antigens codiert, und eine Nukleinsäure, die ein Polypeptid eines MUC-1-Antigens codiert, umfasst; und (b) wenigstens einen von einem Antagonisten von PD-1, bei dem es sich um einen Antikörper handelt, und einem Antagonisten von LAG-3, bei dem es sich um einen Antikörper handelt; wobei (a) und (b) als Kombination zu verabreichen sind.

2. Arzneimittel oder Zusammensetzung zur Verwendung nach Anspruch 1, wobei das rekombinante Orthopoxvirus ausgewählt ist aus einem Vacciniavirus, einem modifizierten Vaccinia Ankara (MVA) und MVA-BN.

3. Arzneimittel oder Zusammensetzung zur Verwendung nach Anspruch 1-2, ferner umfassend: (c) ein rekombinantes Avipoxvirus, das eine Nukleinsäure, die ein Polypeptid wenigstens eines Tumorantigens codiert, umfasst; und (d) wenigstens einen von einem Antagonisten von PD-1, bei dem es sich um einen Antikörper handelt, und einem Antagonisten von LAG-3, bei dem es sich um einen Antikörper handelt; wobei (c) und (d) als Kombination zu verabreichen sind.

4. Arzneimittel oder Zusammensetzung zur Verwendung nach Anspruch 3, wobei die Kombination rekombinantes Avipoxvirus und Antagonist nach der Kombination rekombinantes Orthopoxvirus und Antagonist nach Anspruch 1 zu verabreichen ist.

5. Arzneimittel oder Zusammensetzung zur Verwendung nach Anspruch 3-4, wobei es sich bei dem Avipoxvirus um ein Hühnerpockenvirus handelt.

6. Arzneimittel oder Zusammensetzung zur Verwendung nach Anspruch 1-2, ferner umfassend: (e) zwei oder mehr rekombinante Orthopoxviren, die eine Nukleinsäure, die ein Polypeptid wenigstens eines Tumorantigens codiert, umfassen; und (f) wenigstens einen von einem Antagonisten von PD-1, bei dem es sich um einen Antikörper handelt, und einem Antagonisten von LAG-3, bei dem es sich um einen Antikörper handelt; wobei (e) und (f) als Kombinationsbehandlung zu verabreichen sind.

7. Arzneimittel oder Zusammensetzung zur Verwendung nach Anspruch 1-6, wobei es sich bei dem Krebs um Brustkrebs, Lungenkrebs, Kolorektalkarzinom, Magenkrebs, Bauchspeicheldrüsenkrebs, Prostatakrebs, Blasenkrebs oder Ovarialkarzinom handelt.

## Revendications

1. Médicament ou composition pour utilisation dans le traitement d'un patient cancéreux humain, comprenant : (a) un premier orthopoxvirus recombinant comprenant un acide nucléique codant pour un polypeptide d'un antigène CEA et un acide nucléique codant pour un polypeptide d'un antigène MUC-1 ; et (b) au moins l'un parmi un antagoniste de PD-1 qui est un anticorps et un antagoniste de LAG-3 qui est un anticorps ; dans lequel (a) et (b) sont destinés à être administrés sous la forme d'une combinaison.

2. Médicament ou composition pour utilisation selon la revendication 1, dans lequel l'orthopoxvirus recombinant est choisi parmi un virus de la vaccine, un virus de la vaccine Ankara modifié (MVA) et MVA-BN.

3. Médicament ou composition pour utilisation selon les revendications 1 à 2, comprenant en outre : (c) un avipoxvirus recombinant comprenant un acide nucléique codant pour un polypeptide d'au moins un antigène tumoral ; et (d) au moins l'un parmi un antagoniste de PD-1 qui est un anticorps et un antagoniste de LAG-3 qui est un anticorps ; dans lequel (c) et (d) sont destinés à être administrés sous la forme d'une combinaison.

4. Médicament ou composition pour utilisation selon la revendication 3, dans lequel la combinaison d'avipoxvirus recombinant et d'antagoniste est destinée à être administrée après la combinaison d'orthopoxvirus recombinant et d'antagoniste selon la revendication 1.

5. Médicament ou composition pour utilisation selon la revendication 3 à 4, l'avipoxvirus étant un virus de la variole aviaire.

6. Médicament ou composition pour utilisation selon la revendication 1 à 2, comprenant en outre : (e) deux ou plus de deux orthopoxvirus recombinants comprenant un acide nucléique codant pour un polypeptide d'au moins un antigène tumoral ; et (f) au moins l'un parmi un antagoniste de PD-1 qui est un anticorps et un antagoniste de LAG-3 qui est un anticorps ; dans lequel (e) et (f) sont destinés à être administrés sous la forme d'un traitement d'association.

7. Médicament ou composition pour utilisation selon les revendications 1 à 6, le cancer étant un cancer du sein, un cancer du poumon, un cancer colorectal, un cancer de l'estomac, un cancer du pancréas, un cancer de la prostate, un cancer de la vessie ou un cancer des ovaires.


### SEQUENCE LISTING

<110> BAVARIAN NORDIC, INC
<120> COMBINATION THERAPY FOR TREATING CANCER WITH A POXVIRUS EXPRESSING A TUMOR ANTIGEN AND AN ANTAGONIST AND/OR AGONIST OF AN IMMUNE CHECKPOINT INHIBITOR
<130> BNIT0007PCT
<150> US 61/900,226
   <151> 2013-11-05
<160> 2
<170> PatentIn version 3.5
<210> 1
   <211> 4487
   <212> DNA
   <213> Artificial Sequence
<220>
   <223> Nucleotide sequence of a construct encoding a modified HER2 protein including two epitopes derived from tetanus toxin
<400> 1
<210> 2
   <211> 683
   <212> PRT
   <213> Artificial Sequence
<220>
   <223> Amino acid sequence of the Modified HER2 protein encoded by SEQ ID NO:1
<220>
   <221> P2
   <222> (273)..(287)
   <223> T-helper cell epitope P2 derived from Tetanus Toxin
<220>
   <221> P30
   <222> (654)..(674)
   <223> T-helper cell epitope P30 derived from Tetanus Toxin
<400> 2
   Met Glu Leu Ala Ala Leu Cys Arg Trp Gly Leu Leu Leu Ala Leu Leu
   1               5                   10                  15
   Pro Pro Gly Ala Ala Ser Thr Gln Val Cys Thr Gly Thr Asp Met Lys
               20                  25                  30
   Leu Arg Leu Pro Ala Ser Pro Glu Thr His Leu Asp Met Leu Arg His
           35                  40                  45
   Leu Tyr Gln Gly Cys Gln Val Val Gln Gly Asn Leu Glu Leu Thr Tyr
       50                  55                  60
   Leu Pro Thr Asn Ala Ser Leu Ser Phe Leu Gln Asp Ile Gln Glu Val
   65                  70                  75                  80
   Gln Gly Tyr Val Leu Ile Ala His Asn Gln Val Arg Gln Val Pro Leu
                   85                  90                  95
   Gln Arg Leu Arg Ile Val Arg Gly Thr Gln Leu Phe Glu Asp Asn Tyr
               100                 105                 110
   Ala Leu Ala Val Leu Asp Asn Gly Asp Pro Leu Asn Asn Thr Thr Pro
           115                 120                 125
   Val Thr Gly Ala Ser Pro Gly Gly Leu Arg Glu Leu Gln Leu Arg Ser
       130                 135                 140
   Leu Thr Glu Ile Leu Lys Gly Gly Val Leu Ile Gln Arg Asn Pro Gln
   145                 150                 155                 160
   Leu Cys Tyr Gln Asp Thr Ile Leu Trp Lys Asp Ile Phe His Lys Asn
                   165                 170                 175
   Asn Gln Leu Ala Leu Thr Leu Ile Asp Thr Asn Arg Ser Arg Ala Cys
               180                 185                 190
   His Pro Cys Ser Pro Met Cys Lys Gly Ser Arg Cys Trp Gly Glu Ser
           195                 200                 205
   Ser Glu Asp Cys Gln Ser Leu Thr Arg Thr Val Cys Ala Gly Gly Cys
       210                 215                 220
   Ala Arg Cys Lys Gly Pro Leu Pro Thr Asp Cys Cys His Glu Gln Cys
   225                 230                 235                 240
   Ala Ala Gly Cys Thr Gly Pro Lys His Ser Asp Cys Leu Ala Cys Leu
                   245                 250                 255
   His Phe Asn His Ser Gly Ile Cys Glu Leu His Cys Pro Ala Leu Val
               260                 265                 270
   Gln Tyr Ile Lys Ala Asn Ser Lys Phe Ile Gly Ile Thr Glu Leu Arg
           275                 280                 285
   Tyr Thr Phe Gly Ala Ser Cys Val Thr Ala Cys Pro Tyr Asn Tyr Leu
       290                 295                 300
   Ser Thr Asp Val Gly Ser Cys Thr Leu Val Cys Pro Leu His Asn Gln
   305                 310                 315                 320
   Glu Val Thr Ala Glu Asp Gly Thr Gln Arg Cys Glu Lys Cys Ser Lys
                   325                 330                 335
   Pro Cys Ala Arg Val Cys Tyr Gly Leu Gly Met Glu His Leu Arg Glu
               340                 345                 350
   Val Arg Ala Val Thr Ser Ala Asn Ile Gln Glu Phe Ala Gly Cys Lys
           355                 360                 365
   Lys Ile Phe Gly Ser Leu Ala Phe Leu Pro Glu Ser Phe Asp Gly Asp
       370                 375                 380
   Pro Ala Ser Asn Thr Ala Pro Leu Gln Pro Glu Gln Leu Gln Val Phe
   385                 390                 395                 400
   Glu Thr Leu Glu Glu Ile Thr Gly Tyr Leu Tyr Ile Ser Ala Trp Pro
                   405                 410                 415
   Asp Ser Leu Pro Asp Leu Ser Val Phe Gln Asn Leu Gln Val Ile Arg
               420                 425                 430
   Gly Arg Ile Leu His Asn Gly Ala Tyr Ser Leu Thr Leu Gln Gly Leu
           435                 440                 445
   Gly Ile Ser Trp Leu Gly Leu Arg Ser Leu Arg Glu Leu Gly Ser Gly
       450                 455                 460
   Leu Ala Leu Ile His His Asn Thr His Leu Cys Phe Val His Thr Val
   465                 470                 475                 480
   Pro Trp Asp Gln Leu Phe Arg Asn Pro His Gln Ala Leu Leu His Thr
                   485                 490                 495
   Ala Asn Arg Pro Glu Asp Glu Cys Val Gly Glu Gly Leu Ala Cys His
               500                 505                 510
   Gln Leu Cys Ala Arg Gly His Cys Trp Gly Pro Gly Pro Thr Gln Cys
           515                 520                 525
   Val Asn Cys Ser Gln Phe Leu Arg Gly Gln Glu Cys Val Glu Glu Cys
       530                 535                 540
   Arg Val Leu Gln Gly Leu Pro Arg Glu Tyr Val Asn Ala Arg His Cys
   545                 550                 555                 560
   Leu Pro Cys His Pro Glu Cys Gln Pro Gln Asn Gly Ser Val Thr Cys
                   565                 570                 575
   Phe Gly Pro Glu Ala Asp Gln Cys Val Ala Cys Ala His Tyr Lys Asp
               580                 585                 590
   Pro Pro Phe Cys Val Ala Arg Cys Pro Ser Gly Val Lys Pro Asp Leu
           595                 600                 605
   Ser Tyr Met Pro Ile Trp Lys Phe Pro Asp Glu Glu Gly Ala Cys Gln
       610                 615                 620
   Pro Cys Pro Ile Asn Cys Thr His Ser Cys Val Asp Leu Asp Asp Lys
   625                 630                 635                 640
   Gly Cys Pro Ala Glu Gln Arg Ala Ser Pro Leu Thr Ser Phe Asn Asn
                   645                 650                 655
   Phe Thr Val Ser Phe Trp Leu Arg Val Pro Lys Val Ser Ala Ser His
               660                 665                 670
   Leu Glu Ile Val Ser Ala Val Val Gly Ile Leu
           675                 680